# NOUVEAU FASCICULE DE BREVET EUROPEEN

(11) **EP 2 922 518 B2**
(45) Date de publication et mention de la décision concernant l'opposition: **06.12.2023**
(45) Mention de la délivrance du brevet: 30.09.2020
(21) Numéro de dépôt: 13805431.7
(22) Date de dépôt: 17.10.2013
(51) Int. Cl.: A61H 7/00, A61H 15/00, A61H 23/02, A61N 5/06, A61N 1/30

(54) **APPAREIL DE MASSAGE EQUIPE DE TÊTES DE MASSAGE INTERCHANGEABLES ET RECONNAISSABLES**
MASSAGEVORRICHTUNG MIT AUSWECHSELBAREN UND IDENTIFIZIERTEN MASSAGEKÖPFEN
MASSAGE DEVICE EQUIPPED WITH INTERCHANGEABLE AND IDENTIFIED MASSAGE HEADS

(30) Priorité: 22.11.2012 FR 1261107
(43) Date de publication de la demande: 30.09.2015
(73) Titulaire: SEB S.A., 69130 Ecully (FR)
(72) Inventeur: GIRAUD, Camille, F-69001 Lyon (FR); PAGET, Monique, F-38460 Optevoz (FR); MANDICA, Franck, F-69340 Francheville (FR)
(74) Mandataire: Poindron, Cyrille
(86) Numéro de dépôt international: PCT/FR2013/052483
(87) Numéro de publication internationale: WO 2014/080103

(56) Documents cités:
- WO-A1-2010/012857
- CN-U- 201 750 912
- GB-A- 1 021 836
- KR-B1- 101 004 373
- US-A- 5 103 809
- US-A1- 2008 209 650
- US-A1- 2011 184 499

## Description

La présente invention concerne le domaine des appareils de traitement de la peau, notamment celle du visage. L'appareil selon l'invention permet, pour le moins, le massage de la peau afin de lui donner de la tonicité. L'appareil de massage selon l'invention trouvera son application chez les ménages composés de personnes désireuses de soigner leur esthétisme en remodelant, raffermissant et rajeunissant leur peau, notamment celle du visage.

Les appareils de massage de la peau se composent généralement d'un corps muni de moyens de motorisation et d'une tête de massage qui comprend des éléments de massage configurés pour être activés sous l'action des moyens de motorisation, par le biais d'un mécanisme de transmission. Parmi l'art antérieur existant, il est notamment connu le brevet EP 1 925 275 B1 qui, outre les caractéristiques précitées, divulgue des moyens d'assemblage et un mécanisme de transmission qui permettent de remplacer facilement la tête de massage pour la changer par une neuve ou différente, la conception du mécanisme de transmission permettant, par ailleurs, d'assembler la tête de massage sur le corps avec un angle d'orientation de l'axe de la tête par rapport à l'axe du corps qui peut être différent.

On connaît également le document US2008/209650 qui divulgue un appareil comprenant un corps motorisé qui permet d'assembler différents types de tête de brosse à dents, le corps pouvant comprendre des moyens de reconnaissance du type de brosse à dents assemblé sur le corps. Il est également indiqué, en variante, qu'une tête de massage peut être assemblée sur le corps motorisé.

L'objet de la présente invention est de concevoir un appareil de massage de la peau qui permet d'assembler différents types de tête de massage sur le corps de l'appareil de massage, tout en optimisant les performances et les caractéristiques de l'appareil de massage en vue de l'adapter au mieux en fonction du type de tête de massage utilisé. A cet effet, l'invention est définie dans les revendications indépendantes 1, 2, 3 et 4.

L'invention concerne un appareil de massage comprenant un corps qui comporte des moyens de motorisation, au moins un type de tête de massage qui comprend au moins deux éléments de massage, un mécanisme de transmission qui permet l'activation des éléments de massage sous l'action des moyens de motorisation, des moyens d'assemblage qui sont configurés pour assembler de manière amovible l'au moins un type de tête de massage avec le corps, des moyens de reconnaissance du type de tête de massage assemblé sur le corps et des moyens de commande dudit appareil de massage qui, en fonction de la reconnaissance du type de tête de massage, sont configurés pour agir sur les moyens de motorisation de sorte à commander le mouvement d'au moins deux éléments de la tête de massage. Lesdits moyens de commande étant configurés pour modifier l'actionnement des moyens de motorisation de sorte à adapter la vitesse d'activation des éléments de massage de la tête de massage.

Ainsi, l'appareil permet de détecter le type de tête assemblée sur le corps et s'adapte en fonction du type de tête de massage, par exemple en adaptant la vitesse des moyens de motorisation, en commandant l'un ou l'autre des éléments des moyens de motorisation, voire autres, pour in fine commander le mouvement des au moins deux éléments de toute tête de massage. Cela présente pour avantage de pouvoir adapter des têtes de massage présentant des caractéristiques de fonctionnement bien différentes entre elles.

Selon l'appareil de massage objet de l'invention, les moyens de commande sont configurés pour agir sur les moyens de motorisation et modifier leur actionnement en fonction du type de tête de massage assemblé sur le corps. Ainsi, par exemple, la vitesse d'activation des éléments de massage de la tête de massage peut être adaptée, ce qui permet d'optimiser les conditions d'utilisation de la tête de massage. Cela permet également d'agencer sur le corps des moyens de motorisation plus ou moins complexes, configurés pour actionner différents types de tête de massage présentant des caractéristiques de conception très diverses et de commander ces moyens de motorisation en fonction de la reconnaissance de la tête de massage.

Selon l'invention, les moyens de commande dudit appareil de massage peuvent être configurés pour agir sur les moyens de motorisation de sorte à commander de façon liée un mouvement desdits au moins deux éléments de massage. Le mouvement est lié car l'actionnement des éléments de massage est entrainé par un même mécanisme de transmission depuis l'arbre moteur des moyens de motorisation, et le mécanisme de transmission agit de façon liée sur chacun des éléments de massage. Le mouvement des au moins deux éléments de massage peut être « lié » mais pas nécessairement identique. Par exemple, comme on le verra ci-dessous, pour la tête appelée pincé Jacquet, les deux éléments de massage rouleaux sont entrainer en rotation autour de leur axe de symétrie dans le même sens mais selon une vitesse différente. Le mouvement entre les au moins deux éléments de massage peut varier par exemple par sa vitesse, son sens, sa direction.

Selon l'invention, les moyens de commande dudit appareil de massage peuvent être configurés pour agir sur les moyens de motorisation de sorte à actionner en rotation chacun desdits au moins deux éléments de massage. Le mouvement peut être différent en ce qu'il est une rotation dans un seul sens, autour d'un axe de symétrie de l'élément par exemple, ou une rotation oscillatoire. Les deux éléments peuvent être en rotation dans le même sens ou dans un sens différent.

Selon l'invention, les éléments de massage sont choisis parmi l'ensemble constitué de : embout de massage, doigt de massage, rouleau de massage, tête de travail, bille de massage. Tous ces éléments sont plutôt en matière rigide (métal, acier, plastique...), ils peuvent être recouverts seulement d'une matière souple type silicone pour mieux accrocher la peau si nécessaire. Tous ces éléments sont proposés pour que l'utilisateur sente sur la peau un appui et un massage ciblé.

Selon l'invention, les moyens de commande dudit appareil de massage sont configurés pour agir sur les moyens de motorisation de sorte à commander au moins un parmi les paramètres suivants desdits au moins deux éléments de massage: la vitesse de rotation, le sens de rotation, la fréquence d'oscillation. La commande peut se faire sur un ou sur plusieurs paramètres en même temps. Plusieurs types de massage sont donc disponibles pour l'utilisateur.

Selon l'appareil de massage objet de l'invention, celui-ci comprend un système ou des moyens d'émission d'ondes et dans lequel les moyens de commande sont configurés pour agir sur le système d'émission d'ondes en fonction du type de tête de massage assemblé sur le corps.

Ces moyens d'émission d'ondes peuvent être agencés sur le corps et l'appareil comprend des moyens de transfert des ondes depuis les moyens d'émission vers la tête de massage assemblée sur le corps. Cela permet de disposer de fonctions de traitement de la peau complémentaires à celles des têtes de massage. Ces ondes peuvent être des ondes électromagnétiques, particulièrement lumineuses, visibles ou dans le domaine de l'infrarouge, ou peuvent être sonores par exemple des ultrasons. Cela permet également de travailler de manière complémentaire, la peau de l'utilisateur. Dans un exemple envisagé de réalisation, ces moyens d'émission d'ondes lumineuses sont parmi au moins une des longueurs d'ondes suivantes : longueurs d'ondes visibles comprenant le rouge (sensiblement 630nm), l'orange (sensiblement 590nm), et longueur d'onde de l'infrarouge (sensiblement 830nm). Dans un mode de réalisation préférentiel mais non limitatif, l'appareil de massage comprend un système luminescent agencé sur le corps et des moyens de transfert de la lumière, du système luminescent vers la tête de massage.

Selon l'appareil de massage objet de l'invention, les moyens de commande sont configurés pour agir sur le système d'émission d'ondes et modifier les ondes en fonction du type de tête de massage assemblé sur le corps. Il peut s'agir de modifier la ou les longueurs d'ondes et / ou l'intensité et /ou la fréquence d'émission des ondes en fonction du type de tête de massage assemblée sur le corps.

Selon l'appareil de massage objet de l'invention les moyens d'émission d'ondes peuvent être agencés sur le corps et des moyens de transfert des ondes, du système d'émission vers la tête de massage. Ceci permet notamment de fournir un appareil compact et économique.

Selon l'invention, les moyens d'émission d'ondes peuvent comprendre des diodes électroluminescentes. L'appareil peut comporter entre 2 et 20 diodes au total, oranges et rouges. L'appareil peut comporter des diodes infrarouges, permettant de produire une chaleur pour obtenir un effet « coup d'éclat ». Ces différentes diodes peuvent être actionnées par type seulement (LED orange seulement, LED rouge seulement, LED infrarouge seulement) pour une tête de massage particulière voire aussi pour une phase de traitement d'une tête particulière.

En effet, selon l'appareil de massage objet de l'invention, les moyens de commande peuvent être configurés pour agir simultanément sur le système d'émission d'ondes et sur les moyens de motorisation de sorte à commander en mouvement au moins deux éléments de la tête de massage, ceci en fonction de la reconnaissance du type de tête de massage pour établir au moins deux phases différentes de traitement de la peau durant le traitement.

Selon un premier mode de réalisation de l'appareil de massage objet de l'invention, les moyens de reconnaissance sont des capteurs mécaniques agencés sur l'au moins un type de tête de massage et le corps et, configurés pour transmettre des informations aux moyens de commande en fonction du type de tête de massage assemblé sur le corps. Les capteurs mécaniques couvrent tout capteur mécanique y compris des contacteurs électriques qui viendraient en contact l'un avec l'autre lorsque la tête de massage est assemblée sur l'appareil.

Selon un second mode réalisation de l'appareil de massage objet de l'invention, les moyens de reconnaissance sont des capteurs magnétiques agencés sur l'au moins un type de tête de massage et le corps et, configurés pour transmettre des informations aux moyens de commande en fonction du type de tête de massage assemblé sur le corps. Ils couvrent tout type de capteur magnétique comme la combinaison ILS avec aimant (ou électroaimant, capteur à effet Hall).

Selon un troisième mode de réalisation de l'appareil de massage objet de l'invention, les moyens de reconnaissance sont des capteurs optiques agencés sur l'au moins un type de tête de massage et le corps et, configurés pour transmettre des informations aux moyens de commande en fonction du type de tête de massage assemblé sur le corps. Ils couvrent tout type de capteur optique comme des photo-capteurs et surfaces optiques, translucides, transparentes, réfléchissantes. Ils sont agencés au fond de la cavité de la partie poignée de l'appareil ou sur ses côtés.

L'appareil ne peut être mis en fonctionnement si aucune tête n'est branchée correctement sur le corps et reconnue par les moyens de reconnaissance.

Selon l'appareil de massage objet de l'invention, celui-ci comprend un système de distribution de produit cosmétique agencé sur le corps et des moyens de transfert du produit, du corps vers la tête de massage. Cela permet également de disposer de fonctions de traitement de la peau complémentaires à celles des têtes de massage. Selon la configuration du système de distribution, la distribution du produit cosmétique pourra être réalisée de manière naturelle, manuelle et/ou automatique.

Dans un mode de réalisation, l'appareil de massage selon l'invention comprend des moyens d'actionnement du système de distribution de produit cosmétique, configurés pour permettre le fonctionnement dudit système de distribution en mode manuel ou en mode automatique. Cela permet de laisser toute liberté d'utilisation de l'appareil de massage ; l'utilisateur peut ainsi choisir de réaliser un traitement cosmétique en complément de celui obtenu avec la tête de massage.

Selon l'appareil de massage objet de l'invention, les moyens de commande sont configurés pour agir sur le système de distribution de produit cosmétique et modifier au moins la distribution du produit cosmétique en fonction du type de tête de massage assemblé sur le corps. Cela permet de réaliser un traitement cosmétique adapté aux différentes têtes de massage.

Selon l'appareil de massage objet de l'invention, les moyens d'assemblage comprennent un système de détection de l'assemblage convenable de la tête de massage sur le corps. Cela permet de garantir une utilisation de l'appareil de massage dans de bonnes conditions.

Selon l'appareil de massage objet de l'invention, celui-ci comprend des moyens de connexion entre les moyens de motorisation et le mécanisme de transmission configurés pour s'affranchir de l'angle d'orientation du mécanisme de transmission par rapport au moyen de motorisation, lors de l'assemblage de la tête de massage sur le corps. Ainsi, il est possible d'assembler des têtes de massage sur le corps avec des orientations différentes, certaines têtes de massage pouvant par exemple être positionnées dans le prolongement du corps lors de l'assemblage, tandis que d'autres têtes sont positionnées avec une inclinaison à 45° par rapport au corps.

Selon un mode de réalisation de l'appareil de massage objet de l'invention, un des types de tête de massage comporte une surface d'application et les éléments de massage sont constitués d'au moins deux embouts de massage qui s'étendent perpendiculairement vers l'extérieur de la surface d'application, les embouts de massage étant définis sur la surface d'application selon des cercles virtuels concentriques de centre C et, dans lequel un mécanisme de transmission est configuré pour rapprocher les embouts de massage et/ou, inversement, pour écarter les embouts de massage, en translatant lesdits embouts de massage dans un sens et/ou dans l'autre selon des trajectoires qui se rejoignent au centre C. Ce type de tête de massage permet de réaliser un pincé de la peau.

Selon un mode de réalisation de l'appareil de massage objet de l'invention, un des types de tête de massage comporte une surface d'application et les éléments de massage sont constitués de deux embouts de massage qui s'étendent perpendiculairement vers l'extérieur de la surface d'application, le mécanisme de transmission étant configuré pour actionner en rotation selon un axe perpendiculaire à la surface d'application, avec un mouvement oscillatoire, les deux embouts. Ce type de tête de massage permet de réaliser un travail avec précision autour des yeux et de la bouche, notamment, en appliquant les embouts de massage perpendiculairement à la peau pour réaliser un effet pincé tournant ou, parallèlement à la peau pour réaliser un effet tenseur remodelant.

Selon un mode de réalisation de l'appareil de massage objet de l'invention, la tête de massage comporte une surface d'application et les éléments de massage sont constitués de trois embouts de massage inclinés vers l'extérieur par rapport à la surface d'application, le mécanisme de transmission étant configuré pour actionner en rotation ou en oscillation, les trois embouts de massage selon trois axes fixes respectifs, perpendiculaires à la surface d'application. Ce type de tête de massage permet de réaliser un léger pincé de la peau.

Selon un mode de réalisation de l'appareil de massage objet de l'invention, la tête de massage comprend une surface d'application et les éléments de massage sont constitués d'au moins deux doigts de massage qui s'étendent vers l'extérieur de la surface d'application, les doigts de massage et le mécanisme de transmission étant configurés pour former une pince. Ce type de tête de massage permet de réaliser un pincé de la peau.

Selon ces divers modes de réalisation de l'appareil de massage objet de l'invention, avec des embouts de massage ou des doigts de massage, la tête de massage comprend une peau souple qui recouvre les éléments de massage. Cela évite que les embouts de massage ou les doigts de massage ne paraissent trop agressifs et cela leur permet de mieux agripper la peau grâce à un contact surfacique sur la peau.

Selon un mode de réalisation de l'appareil de massage objet de l'invention, la tête de massage comprend une couronne s'étendant perpendiculairement à la surface d'application et, le mécanisme de transmission est configuré pour entraîner la couronne avec un mouvement oscillatoire autour d'un axe perpendiculaire à la surface d'application. Cela permet de bien agripper la peau et de réaliser des mouvements complexes sur la peau, en combinaison avec des embouts de massage ou des doigts de massage.

Selon un mode de réalisation de l'appareil de massage objet de l'invention, la tête de massage comprend une surface d'application et les éléments de massage sont constitués de deux rouleaux de massage agencés selon deux axes longitudinaux parallèles, avec un écartement entre eux, et dépassant partiellement à l'extérieur de la surface d'application et, dans lequel un mécanisme de transmission est configuré pour entraîner en rotation de manière synchronisée et inversée, les deux rouleaux de sorte que, dans une vue en plan perpendiculaire aux deux axes, la partie du rouleau de massage située à gauche qui dépasse de la surface d'application, tourne dans le sens trigonométrique et, la partie du rouleau de massage située à droite qui dépasse de la surface d'application, tourne dans le sens horaire. Ce type de tête de massage permet de réaliser un plissage de la peau.

Selon l'invention, l'appareil de massage peut comprendre une tête de massage qui comprend deux éléments de massage constitués de deux rouleaux de massage parallèles qui sont mobiles en rotation sur eux-mêmes selon deux axes de rotation parallèles entre eux et à la surface d'application, distants l'un de l'autre en étant séparés par une zone de travail, un premier rouleau comprenant au moins une palette qui s'étend radialement en saillie de la surface du premier rouleau , et le deuxième rouleau présentant une surface de type lisse, le mécanisme de transmission permettant l'entrainement des rouleaux en rotation et dans le même sens, le premier rouleau allant de l'extérieur de la zone de travail vers l'intérieur de la zone de travail, et le deuxième rouleau allant de l'intérieur de la zone de travail vers l'extérieur de la zone de travail, vu depuis l'extérieur de la tête de massage. Cette tête permet d'automatiser le geste manuel des professionnels de l'esthétique appelé le « pincé Jacquet ».

Selon l'invention, l'appareil de massage peut comprendre une tête de massage (1) qui comprend un élément d'appui destiné à venir contre le visage et qui définit une surface d'appui, au dessus de l'élément d'appui au moins un élément de massage constitué d'un doigt de massage chacun comprenant une tête de travail destinée à venir au contact du visage et chacun étant mobile entre : une position de rétraction (R) dans laquelle la tête de travail est située en deçà de la surface d'appui vers l'intérieur de la tête de massage, une position d'extension (E) dans laquelle la tête de travail est située au delà de la surface d'appui vers l'extérieur de la tête de massage, des moyens de manœuvre de chaque doigt de massage à lier au mécanisme de transmission et adaptés pour déplacer chacun des doigts de massage entre ses positions d'extension (E) et de rétraction (R) de manière alternative. Cette tête permet d'automatiser le geste manuel des professionnels de l'esthétique appelé le tapotement.

Pour cette tête de massage par tapotement, lorsque l'appareil est équipé des moyens d'émission d'ondes, les moyens de commande peuvent être configurés, à la reconnaissance de la tête, pour agir sur le système d'émission d'ondes lumineuses visibles orange. Cette combinaison tapotement-lumière orange permet de fournir une action sur l'épiderme de surface de la peau.

Selon l'invention, l'appareil de massage peut comprendre une tête de massage qui comprend un élément d'appui qui est destiné à venir contre le visage et qui forme une couronne d'appui définissant, d'une part, une surface d'appui s'inscrivant dans un plan d'appui et, d'autre part, une zone de travail située à l'intérieur de la couronne d'appui, à l'intérieur de la couronne d'appui et dans la zone de travail, au moins un élément de massage constitué d'une tête de travail qui possède une surface de travail s'étendant en saillie du plan d'appui, des moyens de manœuvre à lier au mécanisme de transmission et adaptés pour déplacer en rotation chaque tête de travail selon au moins un axe de rotation (Δ, Δ') décalé par rapport au centre de la surface de travail correspondante.

Pour cette tête de massage par une zone de travail mobile à l'intérieur d'une couronne d'appui, lorsque l'appareil est équipé des moyens d'émission d'ondes, les moyens de commande sont configurés pour, à la reconnaissance de la tête, agir sur le système d'émission d'ondes lumineuses infrarouges. Cette combinaison tapotement-lumière orange permet de fournir une action en profondeur dans le derme.

Selon l'appareil de massage objet de l'invention, celui-ci comprend un dispositif de traitement ionophorèse transcutanée qui est configuré pour transmettre sur la peau lors de l'application dudit appareil de massage, un courant permettant d'augmenter et/ou d'accélérer la pénétration d'un produit cosmétique qui peut être soit distribué par le système de distribution de produit cosmétique décrit ci-dessus, soit appliqué directement sur la peau par l'utilisateur. L'ionophorèse est un traitement qui a été développée initialement pour l'application dans la peau de médicaments, en particulier pour de la médecine sportive, mais est également envisagée maintenant pour une meilleure pénétration d'un cosmétique. Dans un mode de réalisation, ce dispositif de traitement ionophorèse transcutanée comprend deux électrodes à potentiel électrique différent qui peuvent être agencées sur des embouts ou rouleaux de massage et/ou sur la surface d'application de la tête de massage. Les réglages de l'appareil de massage selon l'invention, en particulier lorsque celui-ci comprend également un système de distribution de produit cosmétique, permettront de réaliser le traitement par ionophorèse transcutanée avant et /ou pendant l'application du produit cosmétique. Ces réglages dépendront de la reconnaissance de la tête de massage assemblée sur le corps

Dans un programme possible « ionophorèse », il est appliqué un cosmétique dédié, manuellement ou par une tête adéquate, puis une tête spécifique à électrodes est branchée, et ensuite l'appareil sera déplacé avec un contact pour un traitement en continu sur l'ensemble de la zone, ou un maintien sur une zone avec un décompte du timer et l'indication à l'utilisateur quand il faut changer.

Selon une caractéristique de l'invention, l'appareil de massage comporte sur son corps une interface homme machine (IHM) comprenant un écran pour notamment afficher au moins une des données suivantes : la durée du traitement (totale, écoulée, restante), les phases du traitement, le nom de la tête reconnue.

L'IHM peut comprendre aussi des boutons de commande (directement sur écran ou actionneurs mécaniques sur le boitier) pour que l'utilisateur puisse régler et choisir une parmi des programmations proposées, pour ajuster le soin selon le souhait de l'utilisateur (dans les limites définies par la reconnaissance automatique). Ceci permet d'ajuster le soin grâce à des réglages.

L'IHM peut donner le choix à l'utilisateur entre 2 programmations différentes une fois la tête de massage branchée correctement et reconnue. L'IHM peut donner le choix à l'utilisateur entre une même programmation avec deux intensités différentes pour une même tête, l'utilisateur choisit alors une intensité forte ou faible du massage, de l'émission d'ondes.

Cette IHM permet également de pouvoir piloter de façon semi-automatique le changement de tête sur l'appareil. Par exemple, un programme peut commencer par une première phase de massage utilisant une première tête et, à la fin de la première phase, l'IHM indique à l'utilisateur de remplacer celle-ci pour entamer une deuxième phase. Ainsi la désignation de la tête est automatique via les moyens de commande et le changement de tête est fait par l'utilisateur, voire ensuite vérifié par l'appareil avant d'entamer la deuxième phase de traitement. Le pilotage est fait par l'unité de commande électronique comportant une temporisation.

Bien entendu, d'autres modes de réalisation des moyens de reconnaissance sont envisageables sur l'appareil de massage, sans sortir du cadre de l'invention.

La description suivante met en évidence les caractéristiques et avantages de la présente invention, laquelle s'appuie sur des figures parmi lesquelles :
- La figure 1 schématise un appareil de massage selon l'invention pouvant recevoir différentes conceptions de têtes de massage ;
- La figure 2 schématise une tête de massage comprenant des rouleaux de massage ;
- La figure 3 schématise une tête de massage comprenant des embouts de massage ;
- La figure 4 schématise un système d'assemblage amovible entre la tête de massage et le corps ;
- Les figures 5 et 6 schématisent une tête de massage comprenant trois embouts inclinés ;
- La figure 7 schématise une couronne sur la tête de massage ;
- La figure 8 schématise une peau souple sur la tête de massage ;
- Les figures 9 et 10 schématisent un mécanisme de transmission entre la tête de massage et le corps ;
- La figure 11 schématise un système de connexion par fibre optique entre la tête de massage et le corps ;
- Les figures 12 et 13 schématisent une variante de tête de massage munie de pinces ;
- Les figures 14 à 16 schématisent des variantes de moyens de reconnaissance de la tête de massage sur le corps ;
- Sur les figures 17 à 20 schématisent des variantes de moyens de reconnaissance de la tête de massage sur le corps par capteurs optiques 167.
- Les figures 21 et 22 schématisent un système de distribution de produit cosmétique ;
- Les figures 23 et 24 schématisent une variante de système de distribution de produit cosmétique ;
- La figure 25 schématise un système vibratoire sur l'appareil de massage ;
- La figure 26 schématise des diodes luminescentes sur la tête de massage ;
- La figure 27 schématise un dispositif de traitement ionophorèse transcutanée sur l'appareil de massage ;
- La figure 28 illustre un système de distribution agencé entre le corps et la tête de massage ;
- Les figures 29 à 36 illustrent des variantes de réalisation de système d'encliquetage entre la tête de massage et le corps ;
- La figure 37 schématise la reconnaissance de différentes têtes de massage lors de leur assemblage sur le corps.
- Les figures 38 à 44 schématisent une tête selon l'invention appelée « tête de tapotement » et ont leurs propres références numériques :

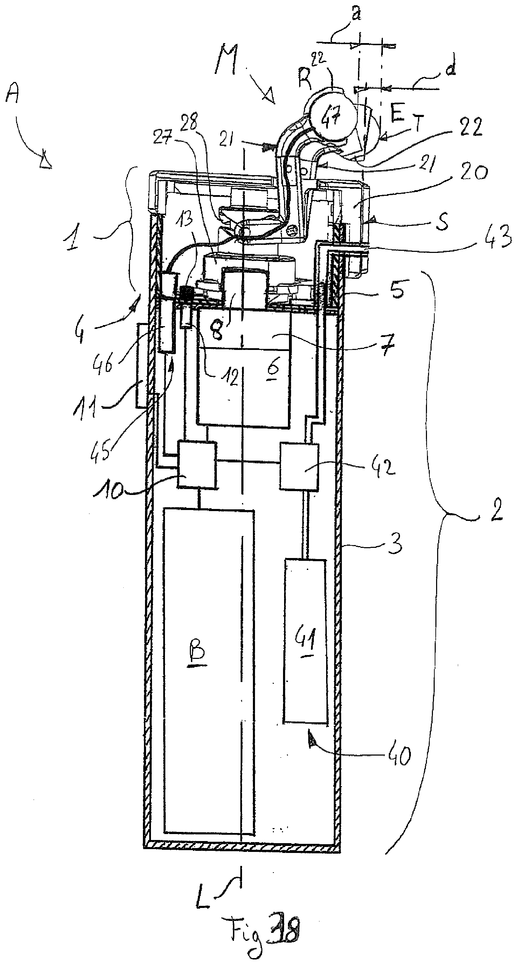

   - La figure 38 est une coupe schématique d'un appareil de massage selon l'invention muni de la tête de tapotement,

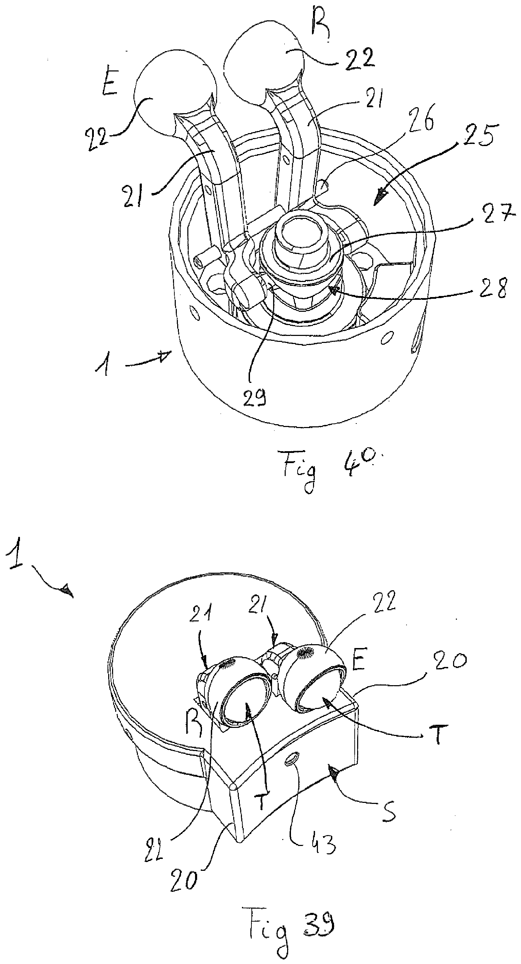

   - La figure 39 est une perspective de la tête de massage amovible équipant l'appareil de massage,
   - La figure 40 est une perspective partiellement arrachée de la tête de massage illustrée à la figure 39,

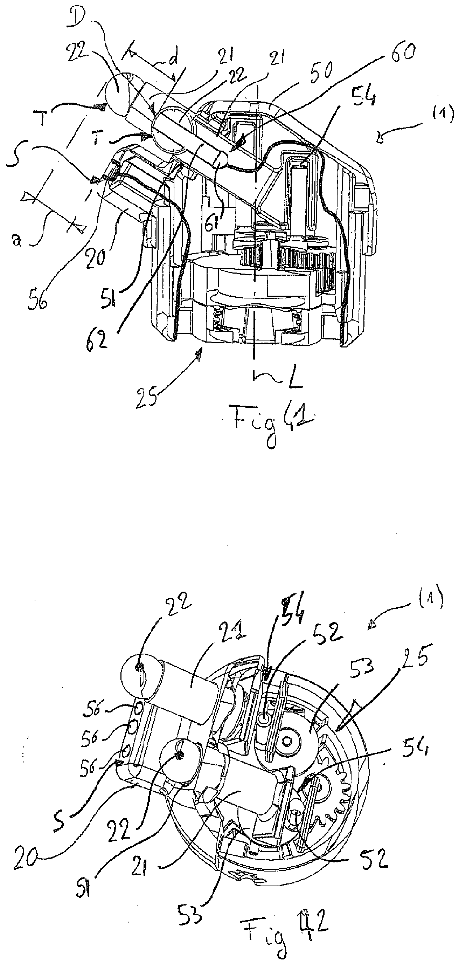

   - La figure 41 est une coupe schématique d'une autre forme de réalisation d'une tête de massage amovible pour un appareil de massage selon l'invention,
   - La figure 42 est une perspective partiellement arrachée de la tête de massage illustrée à la figure 41,

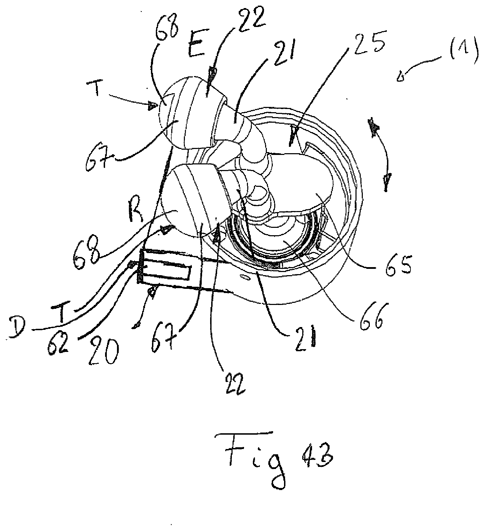

   - La figure 43 est une perspective partiellement arrachée d'une autre forme de réalisation de la tête de tapotement amovible pour un appareil de massage selon l'invention,

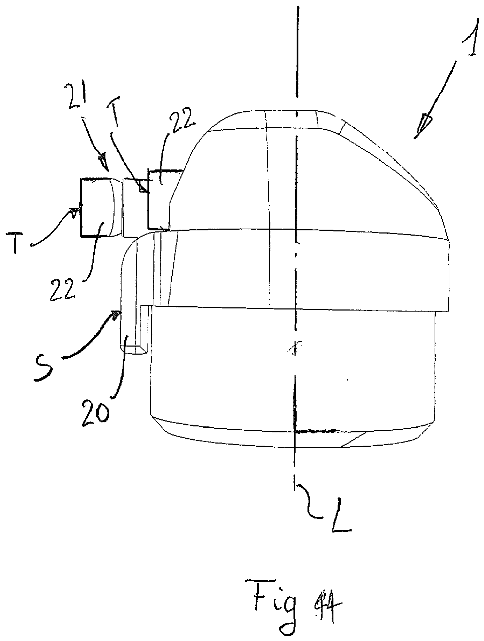

   - La figure 44 est une élévation en vue de côte schématique d'une autre forme de réalisation d'une tête de tapotement amovible pour un appareil de massage selon l'invention.

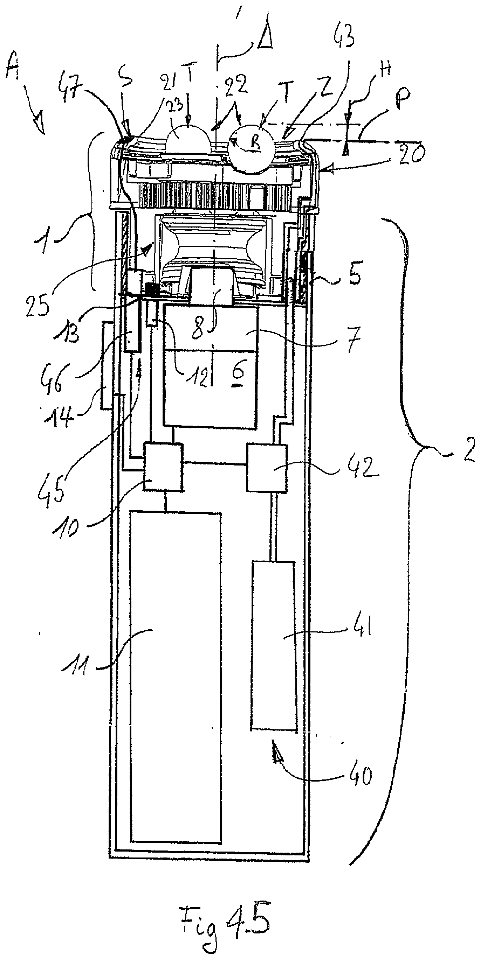

- Les figures 45 à 49 schématisent une tête selon l'invention appelée « tête de zone de travail intérieure» et ont leurs propres références numériques :
   - La figure 45 est une coupe schématique d'un appareil de massage selon l'invention muni de cette tête de zone de travail intérieure,

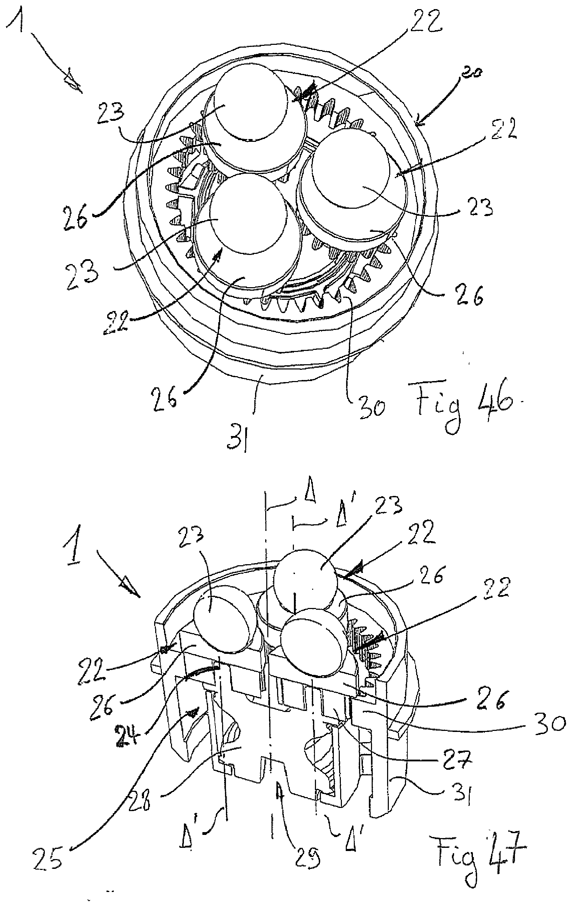

   - La figure 46 est une vue en perspective d'une première tête de zone de travail intérieure,
   - La figure 47 est une coupe de la tête de zone de travail intérieure de la figure 46,

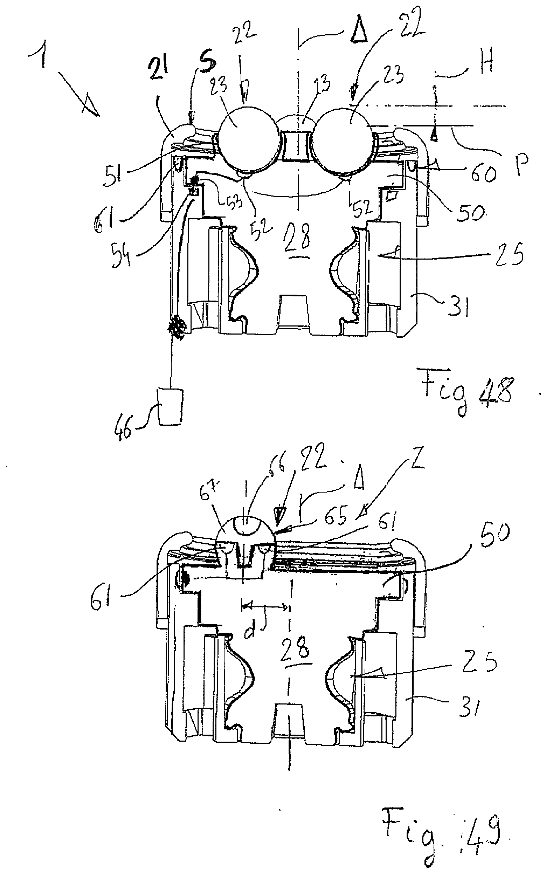

   - La figure 48 est une coupe d'une deuxième tête de zone de travail intérieure,
   - La figure 49 est une coupe d'une troisième tête de zone de travail intérieure,

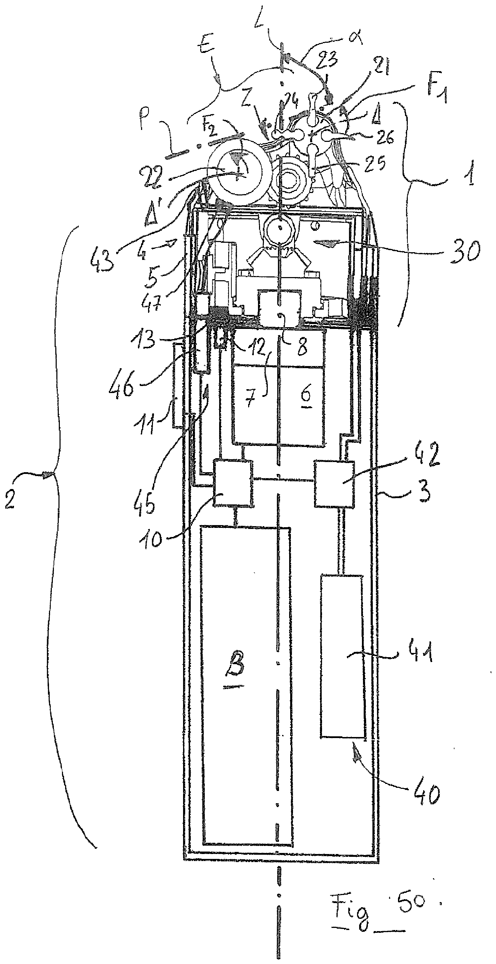

- Les figures 50 à 56 schématisent une tête selon l'invention appelée « tête pincé Jacquet » et ont leurs propres références numériques :
   - La figure 50 est une coupe schématique d'un appareil de massage muni de la tête pincé Jacquet,

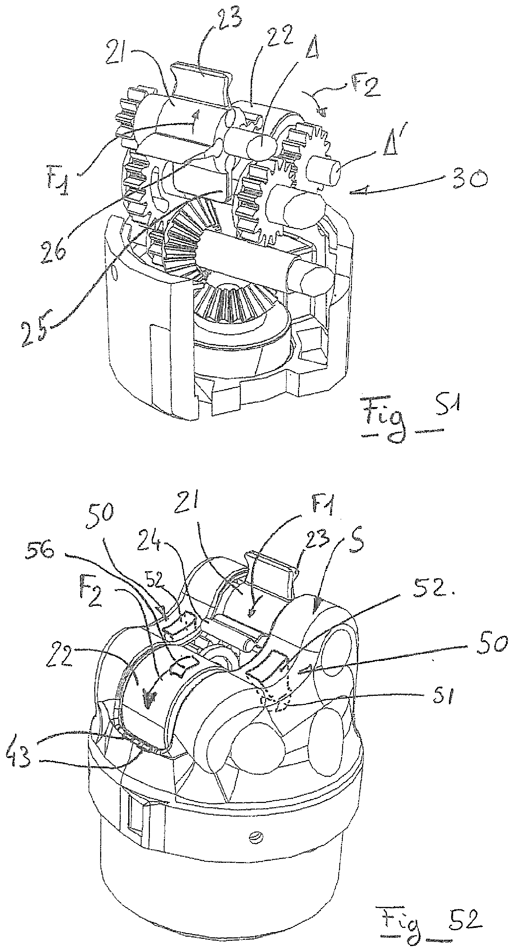

   - La figure 51 est une perspective partiellement arrachée de la tête de massage amovible équipant l'appareil de massage illustré à la figure 50,
   - La figure 52 est une perspective de la tête de massage illustrée à la figure 51,

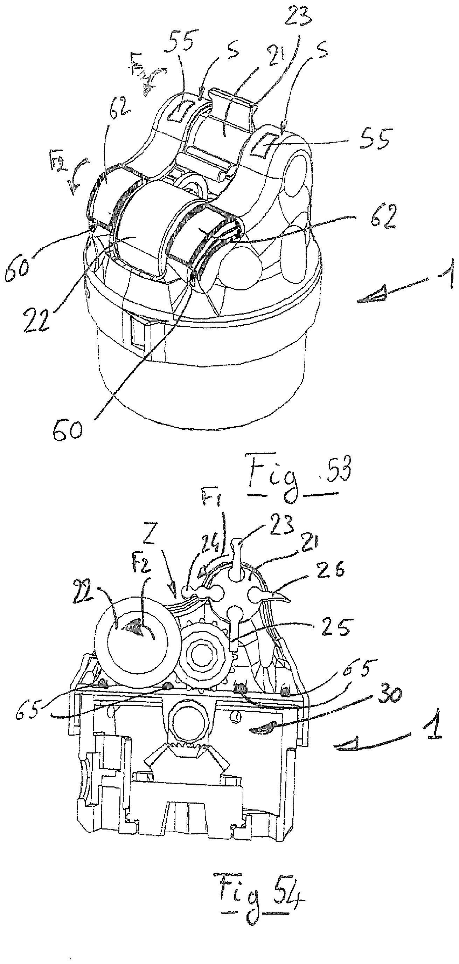

   - La figure 53 est une perspective d'une autre forme de réalisation d'une tête de massage amovible pour un appareil de massage selon l'invention,
   - La figure 54 est une coupe schématique de la tête de massage illustrée à la figure 53,

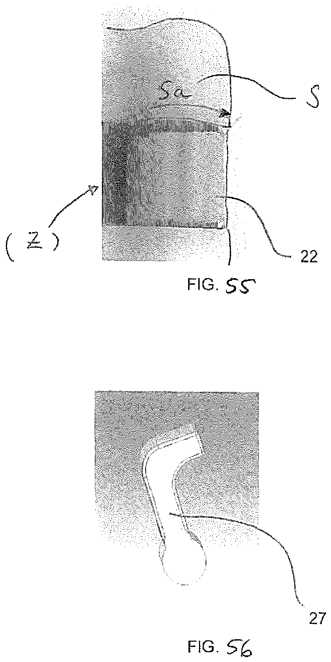

   - La figure 55 est une perspective d'un mode alternatif des figures 50 à 54 de réalisation d'un rouleau de surface de type lisse selon l'invention illustrée,
   - La figure 56 est une perspective d'un mode alternatif des figures 50 à 54 de palette du rouleau selon l'invention.

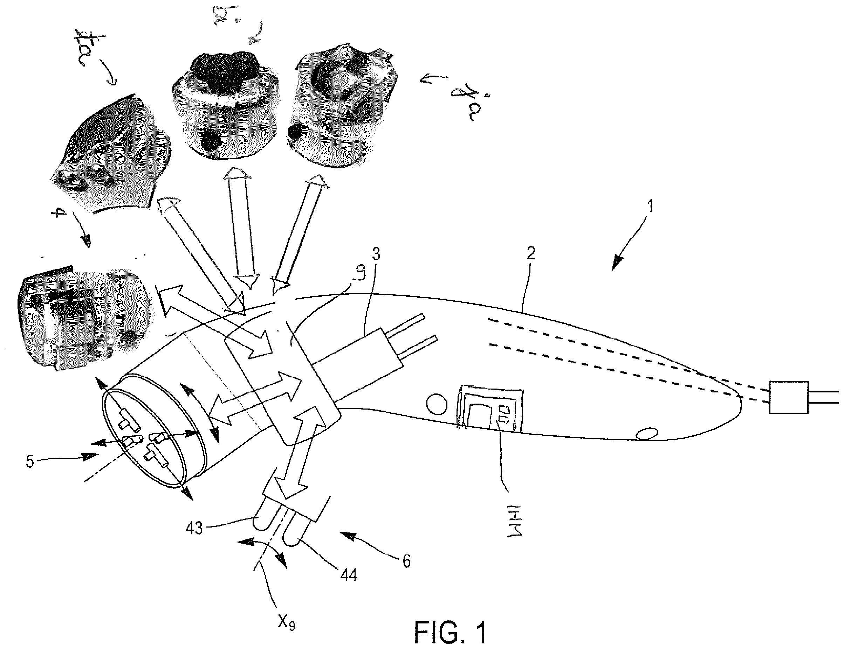

Tel qu'illustré sur la figure 1, l'appareil de massage 1 comprend un corps 2 à l'intérieur duquel est agencé un moteur 3 alimenté électriquement par raccordement soit à une source électrique externe, au moyen d'une prise électrique et d'un transformateur basse tension, soit à une source électrique interne du type batterie rechargeable ou piles jetables. L'appareil de massage 1 comprend également un moto réducteur agencé en sortie du moteur 3 pour réduire sa vitesse de rotation et l'adapter au besoin d'utilisation. L'appareil de massage 1 est configuré pour permettre la connexion de différents types de tête de massage 4, 5, 6, 7, 8, ta, bi et ja illustrés sur les figures. Pour cela, l'appareil de massage comprend des moyens de connexion amovible 9 entre le corps 2 et la tête de massage 4, 5, 6, 7, 8, ta, bi, ja. L'appareil de massage comporte également sur son corps une interface homme machine IHM comprenant un écran pour notamment afficher au moins une des données suivantes : la durée du traitement (totale, écoulée, restante), les phases du traitement, le nom de la tête reconnue. L'IHM peut comprendre aussi des boutons de commande (sur écran ou mécanique sur le boitier) pour que l'utilisateur puisse régler et choisir une parmi des programmations proposées, pour ajuster le soin selon le souhait de l'utilisateur (dans les limites définies par la reconnaissance automatique). Cette IHM permet également de pouvoir piloter de façon semi-automatique le changement de tête sur l'appareil. Par exemple, un programme peut commencer par une phase utilisant une première tête et à la fin de la première phase l'IHM indique à l'utilisateur de remplacer celle-ci pour entamer une deuxième phase. Ainsi la désignation de la tête est automatique via les moyens de commande et le changement de tête est fait par l'utilisateur, voire ensuite vérifié par l'appareil avant d'entamer la deuxième phase de traitement.

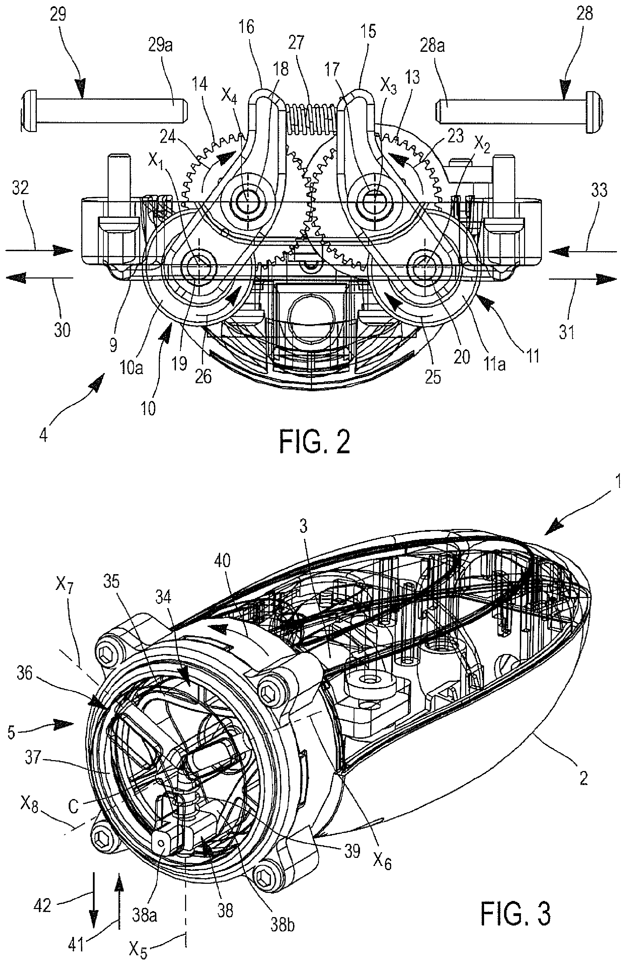

Sur la figure 2, la tête de massage 4 comprend une surface d'application 9 et deux rouleaux de massage 10, 11 dont une partie 10a, 11a dépasse de la surface d'application 9. Ces rouleaux 7, 8 sont disposés selon deux axes X1, X2 parallèle entre eux. Selon ce mode de réalisation, le moteur 3 comprend un arbre d'entraînement assujetti à une roue dentée 13. Ainsi le moteur 3 entraîne en rotation la roue dentée 13 selon un axe X3. Cette roue dentée 13 engrène avec une autre roue dentée 14 de conception identique et disposée selon un axe X4.

Tel qu'illustré sur la figure 2, la tête de massage 4 comprend deux potences 15, 16. Ces potences 15, 16 sont montées en liaison pivot d'axes respectifs X3, X4 au moyen de deux arbres 17, 18. Ces potences reçoivent en liaison pivot d'axe X1, X2 les rouleaux 10, 11 au moyen de deux arbres 19, 20 montés en liaison pivot sur lesdites potences 15, 16.

Cette tête de massage 4 comprend deux autres roues dentées de conception identique (non illustrées). L'une des roues dentées est assujettie au rouleau 11 par le biais de l'arbre 20, par exemple au moyen de cannelures (non illustrées) agencées entre ces éléments, et engrène avec la roue dentée 13. De même, l'autre roue dentée est assujettie au rouleau 10 par le biais de l'arbre 19 et, engrène avec la roue dentée 14. Ainsi, lors de l'entraînement de la roue dentée 13 par le biais du moteur 3, qui tourne dans le sens de la flèche 23, la roue dentée 14 tourne dans le sens de la flèche 24, ce qui permet d'entraîner le rouleau 10 est entraîné dans le sens de la flèche 26 et le rouleau 11 dans le sens de la flèche 25.

Tel qu'illustré sur la figure 2, des ressorts 27 sont agencés entre les deux potences 15, 16. En position initiale, ces ressorts 27 sont actifs contre les potences 15, 16 et les maintiennent en appui contre les extrémités 28a, 29a de deux vis de réglage 28, 29 agencées sur la tête de massage 4. Ainsi les rouleaux 10 ou 11 sont écartés l'un de l'autre avec un écartement minimal en position initiale. En outre, le réglage des vis 28, 29 permet de modifier l'écartement minimal entre ces deux rouleaux 7, 8. Lorsque les parties 10a, 11a des rouleaux 10, 11 sont appliquées sur la peau, les sens des rotations inversés des rouleaux 10, 11 dans le sens des flèches 25, 26 permet de former un pli sur la peau. Les efforts exercés par la peau sur ces rouleaux, lorsque ces efforts sont supérieurs aux efforts exercés par les ressorts 27, permettent alors de comprimer ces ressorts 27 et d'écarter les rouleaux dans le sens des flèches 30, 31 illustrées en figure 2. Au contraire, si ces efforts exercés par la peau sur les rouleaux 10, 11 sont inférieurs aux efforts exercés par les ressorts 27, ces ressorts 27 se détendent et ramènent les rouleaux 10, 11 dans le sens des flèches 32, 33 illustrées en figure 2, jusqu'à atteindre une position d'équilibre.

Des variantes de réalisation de mécanismes de transmission sur la tête d'assemblage 4, fonctionnant selon le même principe, sont envisageables sans sortir du cadre de l'invention.

Tel qu'illustré en figure 3, la variante de tête de massage 5 comprend une pièce de transmission 34 qui comporte une came 35 de forme triangulaire. Une pièce de guidage 36, de forme cylindrique, présente une surface d'application 37 qui est positionnée parallèlement à la surface de la peau lors de l'utilisation de l'appareil de massage 1.

Sur cette figure 3, est illustré un seul embout de massage 38. On comprend toutefois en regard de cette figure 3, que la tête de massage 5 selon ce mode de réalisation comprend trois embouts de massage uniformément répartis et identiques. L'embout de massage 38 comprend une portion externe 38a qui s'étend perpendiculairement à la surface d'application 37, vers l'extérieur de celle-ci. Cette portion externe 38a vient en contact avec la peau de l'utilisateur lors de l'application de l'appareil de massage 1.

Tel qu'illustré sur la figure 3, la pièce de guidage 36 permet de monter en translation selon l'axe X5 sur la pièce de guidage 36. Il en est de même pour les embouts selon les axes X6 et X7. Le moteur 3 permet l'entraînement en rotation selon l'axe X8 de la pièce de guidage 36.

Tel qu'illustré en figure 3, l'embout de massage 38 comprend une portion interne 38b. Lorsque la tête de massage 5 est assemblée sur le corps 2, cette portion interne 38b est positionnée à l'intérieur de la came 35 de la pièce de transmission 9. Cette portion interne 38b est maintenue en appui contre la came 35 au moyen d'un ressort 39. Lorsque la pièce de guidage 36 est entraînée en rotation selon l'axe X8 dans le sens de la flèche 40, l'embout de massage 38 tourne avec celle-ci. La came 35 exerce un effort contre la portion interne 69 et pousse l'embout de massage 38 dans le sens de la flèche 41 sur une première portion angulaire, ce qui permet de translater l'embout de massage 21 selon l'axe X5 dans le sens de la flèche 41, vers le centre C défini par l'intersection de l'axe X8 avec les axes X5, X6, X7. Puis, sur une seconde portion angulaire, la came 35 cesse d'exercer cet effort sur la portion interne 38b. Le ressort 39 qui exerce une fonction de retour, pousse contre de l'embout de massage 38 dans le sens de la flèche 42 et le translate selon l'axe X5 dans le sens de cette flèche 42, pour l'éloigner du centre C. La rotation de la pièce de guidage 36 autour de l'axe X8 combiné à la translation de l'embout de massage 38 selon l'axe X5 permet donc à cet embout de massage 38 de se rapprocher puis de s'éloigner du centre C en effectuant un mouvement de translation circulaire ou curviligne. On comprend que les deux autres embouts de massage subissent les mêmes mouvements en synchronisation avec l'embout de massage 38. Ainsi les trois embouts de massage 38 sont disposés sur un même cercle virtuel de centre C et se déplacent en effectuant une translation curviligne passant par le centre C. Cela permet de réaliser un pincé tournant de la peau au moyen de la tête de massage 5.

Des variantes de réalisation à quatre embouts sont envisageables pour la tête de massage 5. On peut également prévoir une pièce guidage fixe et une pièce de transmission tournant autour de l'axe X8, dans quel cas les embouts de massage translateront radialement vers le centre C pour s'éloigner ou se rapprocher de celui-ci. On peut également envisager de multiples variantes de cames en fonction du nombre d'embouts présent.

D'autres variantes de têtes de massage sont envisageables. Sur la figure 1 on constate que la tête de massage 6 comprend deux embouts 43, 44 qui sont entraînés en rotation ou en oscillation autour d'un axe X9 par le biais du moteur 3 et d'un mécanisme de transmission.

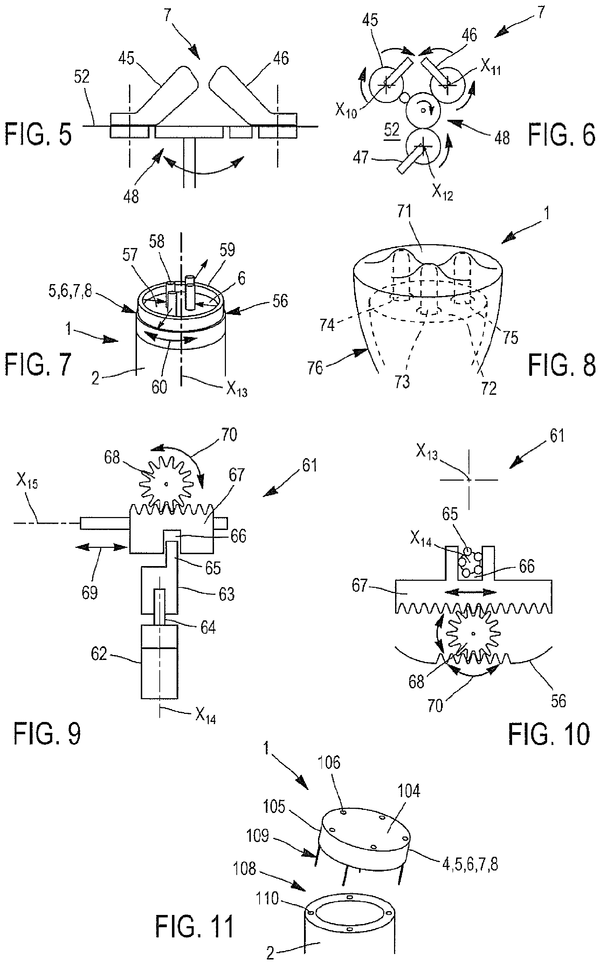

Sur les figures 5 et 6, la tête de massage 7 comprend trois embouts de massage 45, 46, 47 entraînés en rotation autour des axes X10, X11, X12 par un mécanisme d'engrenage 48. Ces embouts de massage 45, 46, 47 sont inclinés par rapport à la surface d'application 52.

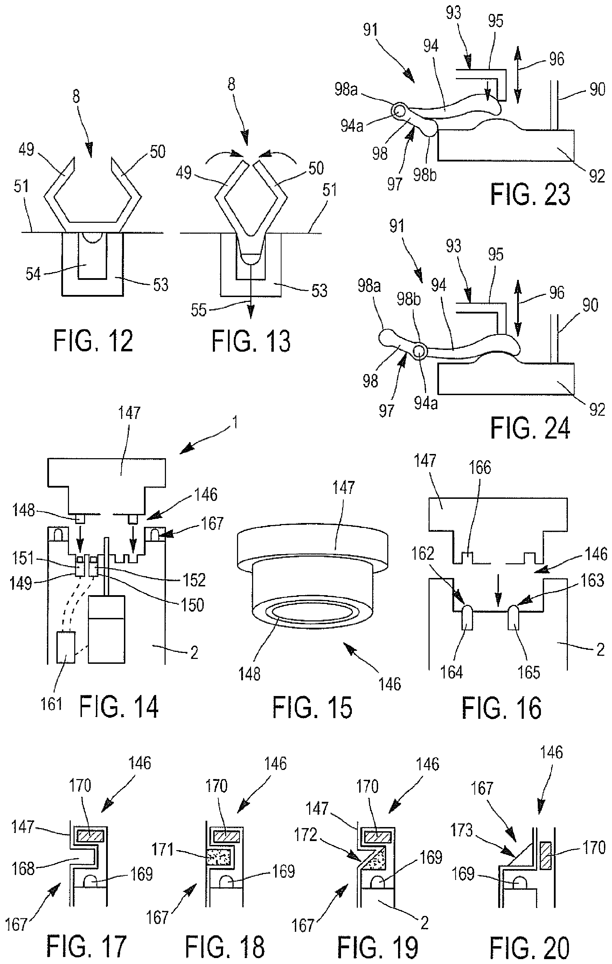

Sur les figures 12 et 13, la tête de massage 8 comprend deux pinces 49, 50 qui s'étendent vers l'extérieur de la surface d'application 51. Ces pinces 49, 50 sont souples. La tête de massage 8 comprend une pièce rigide 53 avec une chambre 54 dans laquelle peuvent pénétrer les pinces 49, 50 qui se déforment du fait de leur souplesse et se ferment. La translation des pinces 49, 50 à l'intérieur de la chambre 54 est assurée par un mécanisme de traction dans le sens de la flèche 55. La souplesse des pinces 49, 50 permet leur retour en position écartée lorsque le mécanisme de traction autorise le déplacement de ces pinces 49,50 dans le sens inversé à la flèche 55.

Dans une variante illustrée sur la figure 7, l'appareil de massage 1 comprend une couronne 56 qui peut être agencée avec les têtes de massage 5, 6, 7, 8 à embouts ou à pinces. Sur la figure 7, des embouts 58 sont représentés. Cette couronne 56 est entraînée en rotation selon l'axe X13 et s'étend par rapport à la surface d'application 57 de sorte que les extrémités des embouts de massage 58 soient positionnées dans le même plan que le bord périphérique 59 de la couronne 56. Cette couronne 56 est de préférence actionnée en rotation selon un mouvement alternatif d'axe X13 dans les sens de la flèche 60. Pour cela, l'appareil de massage 1 comprend un mécanisme de transmission 61 illustré en figure 9 et 10. Ce mécanisme de transmission 61 comprend un moto réducteur 62 qui est disposé dans le corps 2 de l'appareil de massage 1. Ce moto réducteur 62 peut être différent ou le même que le moto réducteur du moteur 3. Il peut en outre être entraîné par le même moteur 3 par le biais d'un système d'engrenage (non illustré) voire par un moteur indépendant disposé dans le corps 2. Un excentrique 63 est agencé sur l'arbre 64 du moto réducteur 62. Cet excentrique 63 comprend un doigt 65 décalé par rapport à l'axe X14 de rotation de l'arbre 64. Ce doigt 65 est positionné dans une rainure 66 agencée sur une crémaillère 67 montée en translation selon un axe X15 et engrenant avec un pignon 68 qui engrène lui-même avec la couronne 56, comme illustré en figures 9 et 10. Ainsi le doigt 65 dispose d'un certain degré de liberté dans la rainure 66 lors de sa rotation autour de l'axe X14, ce qui permet de transmettre un mouvement alternatif selon la flèche 69 à la crémaillère et donc, un mouvement de rotation alternatif selon la flèche 70 au pignon 68 et à la couronne 56. L'avantage de ce mécanisme transmission 61 est de pouvoir modifier facilement l'angle d'inclinaison entre le doigt 65 et la rainure 66, ce qui permet d'assembler la tête de massage sur le corps 2 avec différentes orientations angulaires, par exemple dans l'axe du corps 2 ou avec une inclinaison à 45 degrés par rapport au corps 2. On obtient ainsi des moyens de connexion qui permettent de s'affranchir des angles entre la tête de massage et le corps. Une telle connexion peut être utilisée avec des variantes de mécanisme de transmission entre la tête de massage et le corps, selon le besoin. On peut également envisager des variantes de connexion qui permettent de s'affranchir de l'angle d'inclinaison entre la tête de massage et le corps, comme par exemple, un système d'engrenage à roue conique ou équivalent.

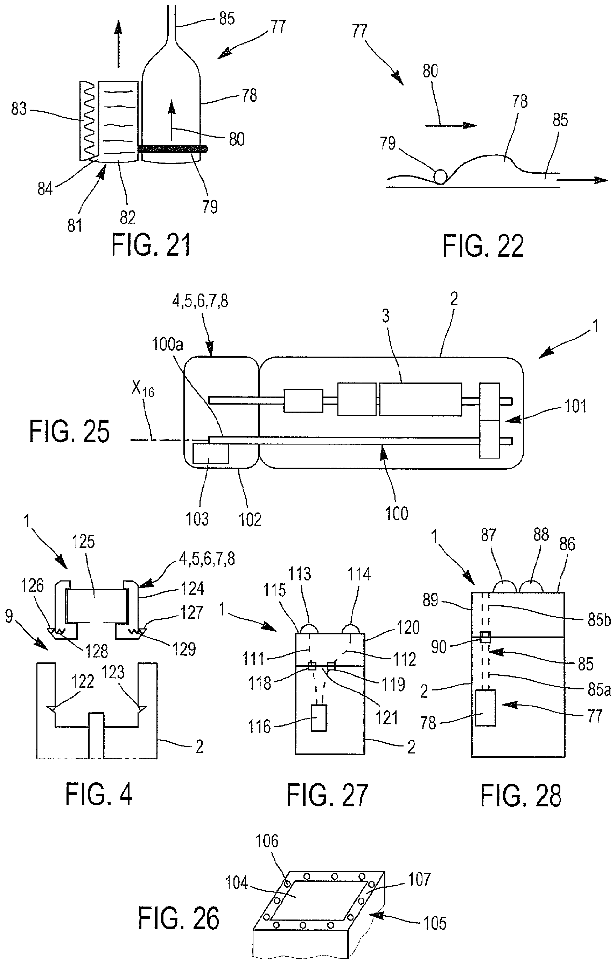

Dans une variante illustrée sur la figure 28, l'appareil de massage 1 comprend une peau souple 71 qui est agencée au-dessus de la surface d'application 72 de la tête de massage 76 et recouvre des embouts de massage 73, 74, 75. Cette peau souple 71 évite notamment que les embouts ne paraissent trop agressifs et améliore l'adhérence sur la peau grâce à un contact surfacique. On peut envisager cette peau souple avec les pinces 49, 50 des figures 12 et 13 ou les diverses variantes à embouts, voire d'autres variantes.

Dans une variante illustrée en figures 21 et 22, on prévoit sur l'appareil de massage 1 un système de distribution de produit cosmétique 77 qui comprend un réservoir de produit 78 souple sur lequel appuie une barrette 79 qui se déplace en translation selon la flèche 80 le long du réservoir de produit 78. Un système d'actionnement 81 permet de déplacer la barrette 79 selon la flèche 80. Ce système d'actionnement 81 est par exemple constitué d'un bouton 82 solidaire de la barrette 79 et translatant dans le sens de cette flèche 80 lors d'une action manuelle. Une pièce crantée 83 permet de maintenir un cran 84 sur le bouton 82 lors de chaque impulsion sur celui-ci pour maintenir en position la barrette 79 au fur et à mesure de son avancement sur le réservoir de produit 78. Le système de distribution 77 comprend un tuyau 85 dont l'agencement permet de distribuer le produit cosmétique au niveau de la surface d'application 86, soit entre les éléments de massage 87, 88, soit au travers des éléments de massage 87, 88, comme illustré en figures 28. Ces éléments de massage 87, 88 sont des rouleaux de massage sur la figure 28 ; ceux-ci peuvent bien entendu être des embouts ou des pinces. On constate sur la figure 28 que le tuyau 85 comprend une première portion 85a disposée dans le corps 2 de l'appareil de massage et, une seconde portion 85b disposée dans la tête de massage 89. Il est prévu des moyens de raccordement étanche 90 entre la première portion 85a et la seconde portion 85b du tuyau. Divers moyens de raccordement étanche 90 pourront être envisagés, par exemple une connexion mâle et femelle munie de joints d'étanchéité.

D'autres variantes de système de distribution de produit cosmétique sont envisageables. On peut notamment prévoir un système d'actionnement automatique permettant de distribuer de manière continue et régulière, par exemple au moyen d'une pompe, par exemple pompe électrique, le produit cosmétique. On peut également prévoir un système de distribution de produit cosmétique de manière naturelle, le produit cosmétique étant diffusé régulièrement grâce à des phénomènes physiques ne nécessitant pas un actionnement extérieur.

Dans une variante illustrée en figures 23 et 24, le système de distribution 91 comprend un réservoir 92 qui est souple et un actionneur 93 qui comprend une manette 94 et un doigt motorisé 95 configuré pour se déplacer selon un mouvement alternatif selon les flèches 96. Le système de distribution 91 comprend un système de glissière 97 qui présente une lumière 98 dans laquelle peut se déplacer l'extrémité arrière 94a de la manette 94. La manette 94 est configurée pour être placée dans une première position illustrée en figure 23, selon laquelle l'extrémité arrière 94a de la manette 94 est positionnée à l'arrière 98a de la lumière et ladite manette 94 est dégagée du doigt motorisé 95. Ainsi, il est nécessaire d'exercer une impulsion manuelle sur la manette 94 pour appuyer sur le réservoir 92 et distribuer du produit cosmétique au travers du tuyau 99 qui pourra présenter une configuration similaire au tuyau 85 sur la figure 28. Au contraire, lorsque l'extrémité arrière 94a de la manette 94 est positionnée à l'avant 98b de la lumière, tel qu'illustré en figure 24, le doigt motorisé 95 actionne automatiquement la manette 94 qui presse le réservoir 92 et distribue le produit cosmétique au travers du tuyau 99. Des variantes de réalisation d'un système de distribution de produit cosmétique avec un système de basculement en mode manuel ou en mode automatique, peuvent être envisagées sans sortir du cadre de l'invention.

Tel qu'illustré en figure 25, l'appareil de massage 1 comprend un arbre 100 qui est entraîné en rotation selon un axe X16 par le moteur 3, au moyen d'un système d'engrenage 101 connu de l'homme du métier. L'extrémité 100a de cet arbre 100 est agencée dans la tête de massage 4, 5, 6, 7, 8, ta, bi, ja, par exemple sur le carter 102 sur les éléments de massage, et reçoit une masselotte 103 qui tourne en balourd autour de l'axe X16 et permet de faire vibrer la tête de massage durant l'application de l'appareil de massage 1. D'autres variantes de système vibratoire restent envisageables. On peut notamment envisager de disposer la masselotte 103 directement dans le corps 2 à proximité de la tête de massage, pour simplifier la conception et éviter la présence d'un système de connexion supplémentaire sur l'arbre 100 au niveau de l'assemblage entre le corps et la tête de massage. On peut également prévoir d'intégrer complètement le système vibratoire dans la tête de massage, un arbre d'entraînement activé par le moteur 3 ou un autre moteur, pénétrant dans la tête, un système de connexion comparable à celui des figures 9 et 10 pouvant alors être envisagé, par exemple.

Dans une variante illustrée en figures 11 et 26, l'appareil de massage 1 comprend au niveau de la surface d'application 104 sur la tête de massage 105, des diodes luminescentes 106 qui sont commandées par un boîtier électronique (non illustré) agencé à l'intérieur du corps 2. Ces diodes luminescentes 106 pourront être allumées soit automatiquement lors de l'actionnement de la tête de massage 4, 5, 6, 7, 8, ta, bi, ja, soit séparément au moyen du bouton de commande distinct (non illustré). Les diodes luminescentes 106 seront disposées par exemple sur le bord périphérique 107 de la surface d'application 104, voire réparties sur ladite surface d'application 104 en dehors des trajectoires des éléments de massage. On pourra utiliser des diodes luminescentes de différentes couleur ou multi-couleurs, selon la longueur d'onde souhaitée et/ou le traitement recherché, voire prévoir un boîtier de commande permettant de modifier la longueur d'onde de ces diodes luminescentes 106. Un système de transmission de la lumière 108 est agencé entre la tête de massage 4, 5, 6, 7, 8, ta, bi, ja et le corps 2. Ce système de transmission de la lumière comprend une connexion par fibres optiques 109, 110 qui permet de diriger la lumière émise vers la surface d'application 104 ou sur les éléments de massage tels que des embouts de massage.

Dans une variante de réalisation illustrée en figure 27, l'appareil de massage 1 comprend deux électrodes 111, 112 qui présentent un potentiel électrique différent. Ces électrodes 111, 112 peuvent consister en deux éléments de massage 113, 114 ou être agencées sur la surface d'application 115. Ces électrodes 111, 112 sont alimentées par une source électrique 116 agencé dans le corps 2. Cette conception permet de réaliser un traitement par ionophorèse transcutanée avant ou pendant l'application d'un produit cosmétique sur la peau, ce qui permet d'accélérer la pénétration du produit cosmétique. Ce produit cosmétique peut être distribué de manière naturelle, manuelle ou automatique par l'appareil de massage 1, voire appliqué directement sur la peau par l'utilisateur. L'appareil de massage 1 comprend des moyens de connexion amovibles 117, 118 qui permettent de déconnecter ou de connecter rapidement les électrodes 111, 112 lors du démontage ou du montage de la tête de massage 120 sur le corps 2. On peut par exemple prévoir des contacteurs électriques au niveau de la zone d'assemblage 121 entre la tête de massage 120 et le corps 2.

Tel qu'illustré au regard des figures 1 et 4, l'appareil de massage 1 comprend des moyens d'assemblage 9 qui sont configurés pour que la tête de massage 4, 5, 6, 7, 8, ta, bi, ja puisse être montée et démontée rapidement du corps 2. Ces moyens d'assemblage 9 sont mis en œuvre au moyen d'un système de fixation amovible entre la tête de massage et le corps. Selon le mode de réalisation sur la figure 4, le corps 2 comprend des encoches 122, 123 et la tête de massage 4, 5, 6, 7, 8, ta, bi, ja comprend une pièce 124 qui reçoit le mécanisme de transmission 125 du mouvement aux éléments de massage, connecté au moteur 3 par le biais d'un système de connexion qui permet de s'affranchir de l'angle d'inclinaison de la tête de massage par rapport au corps2. Cette pièce 124 comprend des crans 126, 127 et des moyens de rétractation 128, 129 des crans 126, 127 permettant de les escamoter pour leur positionnement dans les encoches 122, 123 et aussi leur retrait de ces encoches 122, 123.

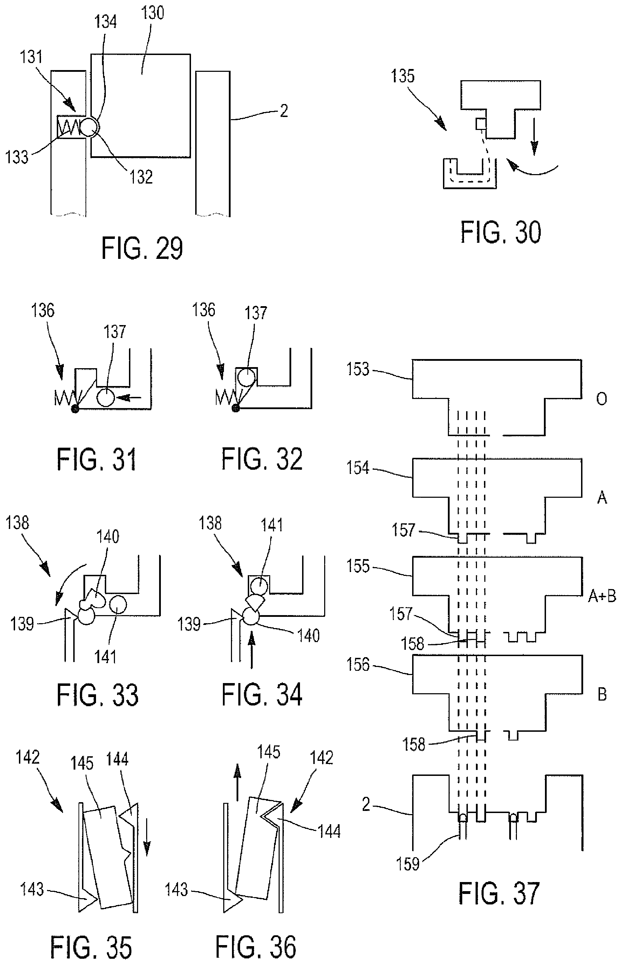

Différents systèmes d'encliquetage ou moyens de rétractation sont illustrés sur les figures 29 à 36 est permettent de maintenir assembler une tête de massage 130 sur le corps 2. Sur la figure 29 un système de butée 131 à bille 132 est illustré, la bille 132 étant montée sur ressort 133 et s'engageant dans une gorge 134 sur la tête de massage 130. Sur la figure 30, un système à baïonnette 135 est illustré. Sur les figures 31 et 32 est illustré un système à clapet et ressort 136 permettant le blocage d'une pièce 137 assujettie à la tête de massage. Sur les figures 33 et 34, un système de blocage 138 avec cran 139 et loquet de déblocage 140 est illustré dans une position de blocage et de déblocage d'une pièce 141. Sur les figures 35 et 36, un système de blocage 142 permet à deux crans 143, 144 de bloquer ou de débloquer une pièce 145. Ces différentes variantes de réalisation ne sont pas limitatives.

Tel qu'illustré en figures 14 à 20, l'appareil de massage 1 comprend des moyens de reconnaissance 146 de la tête de massage 147 assemblée sur le corps 2.

Sur les figures 14 et 15, la tête de massage 146 comprend une protubérance 148 de forme circulaire qui est prévue pour être engagée dans une rainure circulaire 149 sur le corps 2. Le corps 2 comprend une seconde rainure circulaire 150. Dans ces rainures circulaires 149, 150 sont agencés des contacteurs mécaniques 151, 152. Lorsque la tête de massage 146 est assemblée sur le corps 2, la protubérance 148 actionne le contacteur mécanique 151. On constate sur la figure 37 quatre têtes de massage 153, 154, 155, 156 qui peuvent être assemblées sur le corps 2. La première tête de massage 153 ne comprend aucune protubérance. La seconde tête de massage 154 comprend une protubérance 157. La troisième tête de massage 155 comprend deux protubérances 157, 158. Et la quatrième tête de massage 156 comprend une protubérance 158. Lors de l'assemblage de ces têtes de massage 153, 154, 155, 156 sur le corps 2, elles actionnent l'un et/ou l'autre des deux contacteurs mécaniques 159, 160, ce qui permet leur reconnaissance en fonction du codage binaire programmé sur un boîtier de commande 161 illustré en figure 14. Ce boîtier de commande 161 est configuré pour contrôler les différents actionneurs présents dans l'appareil de massage 1 en fonction du type de tête de massage assemblée. Ainsi, par exemple, le boîtier de commande 161 permet de contrôler l'actionnement des moteurs, des pompes de distribution de produit cosmétique, du dispositif de traitement ionophorèse transcutanée, du système vibratoire, du système luminescent...

Sur la figure 16, on constate que les protubérances 162, 163 sont disposées sur le corps 2 et mises en œuvre par les contacteurs mécaniques 164, 165. Tandis que la tête de massage 147 comprend une rainure circulaire 166.

Des variantes sont envisageables dans le cadre de l'invention. On peut notamment remplacer les contacteurs mécaniques par des contacteurs ou des capteurs magnétiques.

Sur les figures 14 et 17 à 20 sont représentés des capteurs optiques 167. Sur la figure 17, la tête de massage 147 comprend une surface transparente 168 qui laisser passer la lumière émise par une diode luminescente 169 et détectée par le capteur optique 170. On détecte ainsi la lumière par transparence. Sur la variante de la figure 18, la surface 171 est opaque, le capteur 170 détecte donc une opacité lors de l'assemblage de la tête de massage 147. Sur la figure 19, un séparateur 172 est agencé entre la tête de massage 147 et le corps 2. Sur la figure 20, un système de miroir est agencé entre la tête de massage 147 et le corps 2.

D'autres variantes sont envisageables sans sortir du cadre de l'invention, notamment quant à la reconnaissance des têtes de massage sur le corps 2 et au boîtier de commande 161. Les capteurs pourront par exemple permettre de détecter l'assemblage convenable de la tête de massage 147 sur le corps 2.

### En ce qui concerne la tête appelée de tapotement, illustrée en figures 38 à 44 avec ses propres références numériques :

Un appareil de massage selon l'invention, tel qu'illustré à la figure 38 et désigné dans son ensemble par la référence A, comprend une tête de massage 1 adaptée de manière amovible sur un boîtier d'entraînement 2. La tête de massage 1 est conçue pour exercer une action mécanique sur la peau du visage d'un utilisateur par l'intermédiaire d'éléments de massage M entraînés par un moteur électrique.

À cet effet, le boîtier d'entraînement 2 comprend un corps allongé 3 de forme généralement cylindrique qui comprend au niveau d'une de ses extrémités 4 des moyens d'adaptation 5 de manière amovible de la tête de massage 1. Les moyens d'adaptation 5 sont, selon l'exemple illustré, formés par un fourreau à l'intérieur duquel la tête de massage 1 est en partie engagée.

Le boîtier d'entraînement 2 comprend, à l'intérieur du corps 3, un moteur électrique 6 qui actionne des moyens d'entraînement 7 adaptés pour transmettre le mouvement du moteur électrique aux éléments de massage de la tête de massage 1. Selon l'exemple illustré, les moyens d'entraînement 7 comprennent un réducteur non représenté qui entraîne un arbre de sortie 8 accessible au niveau des moyens d'adaptation 5 du boîtier d'entraînement 2.

Le moteur électrique 6 est piloté par une unité de commande 10 alimentée par un bloc de batterie B disposé à l'intérieur du corps 3. Bien entendu, l'alimentation électrique de l'unité de commande 10 pourrait également être effectuée directement à partir du réseau électrique par l'intermédiaire d'un transformateur. L'unité de commande 10 est en outre raccordée à une interface de commande manuelle 11 accessible depuis l'extérieur du corps 3. L'interface de commande manuelle 11 peut par exemple comprendre un interrupteur marche-arrêt et/ou des moyens de sélection manuelle de programmes de fonctionnement.

Le boîtier d'entrainement 2 comprend aussi des moyens de reconnaissance 12 qui sont raccordés à l'unité de commande 10 et qui sont adaptés pour lire des moyens d'identification 13 portés par la tête de massage 1. L'unité de commande 10 est alors adaptée pour contrôler le fonctionnement de l'appareil de massage A en fonction de la tête de massage 1 telle que reconnue suite à la lecture des moyens d'identification 13. Le contrôle du fonctionnement de l'appareil de massage A peut notamment consister en une détermination de la vitesse de rotation du moteur électrique 6 de manière qu'elle soit adaptée au massage devant être réalisé par les éléments de massage M. Les moyens d'identification 13 peuvent, par exemple, être constitués par une puce RFID tandis que les moyens de reconnaissance 12 seront adaptés pour lire une telle puce RFID. Bien entendu, les moyens d'identification 13 et de reconnaissance 12 pourraient être réalisés de toute autre manière appropriée comme par exemple sous la forme d'un système d'identification par contact mécanique ou électrique ou encore sous la forme d'un système d'identification magnétique mettant en œuvre des aimants permanents et des interrupteurs à lame souple (ILS) également appelés interrupteurs Reed.

Selon l'invention, la tête de massage 1 est conçue pour réaliser un massage par tapotement. À cet effet, la tête de massage 1 comprend, comme le montre la figure 39 un élément d'appui 20 qui est destiné à venir contre le visage pour définir une distance de travail. Ainsi, l'élément d'appui 20 définit une surface d'appui S qui forme une surface de référence.

Selon l'exemple illustré la surface d'appui S est lisse et présente une forme concave qui lui permet de bien épouser la conformation des pommettes lorsque le massage est réalisé sous les yeux ou autour des yeux.

La tête de massage 1 comprend au-dessus de la surface d'appui S et à l'opposé du boîtier d'entraînement 2 par rapport à ladite surface d'appui S, les éléments de massage M qui comprennent au moins deux doigts de massage 21 qui comprennent chacun une tête de travail 22 destinée à venir au contact du visage.

Les deux doigts de massage 21 sont chacun mobiles entre, d'une part, une position de rétraction R correspondant à la position du doigt de massage 21 situé au premier plan à la figure 39 et, d'autre part, une position d'extension E correspondant à la position du doigt de massage 21 situé au second plan à la figure 39.

En position de rétraction R, la tête de travail 22 de chaque doigt de massage 21 se trouve en deçà de la surface d'appui S vers l'intérieur de la tête de massage 1. Tandis qu'en position d'extension E, la tête de travail 22 de chaque doigt de massage 21 se trouve au-delà de la surface d'appui S vers l'extérieur de la tête de massage 1. Chaque tête de travail 22 possède alors entre ses positions de rétraction R et d'extension E, une amplitude de déplacement a comprise entre 5 mm et 15 mm. Par ailleurs, en position d'extension E chaque tête de travail 22 est saillante par rapport à la surface d'appui S d'une distance d comprise 2 mm et 10 mm.

La tête de massage 1 comprend également des moyens de manœuvre 25 adaptés pour déplacer alternativement chacun des doigts de massage 21 entre ses positions d'extension E et de rétraction R. Les moyens de manœuvre 25 sont alors adaptés pour coopérer avec les moyens d'entraînement 7 et plus particulièrement avec l'arbre de sortie 8 de manière à transmettre et transformer le mouvement de rotation du moteur électrique 6 en un mouvement alternatif des doigts de massage 21.

Selon l'exemple illustré, chaque doigt de massage 21 présente une forme générale en S et est porté par un axe d'articulation 26 situé en partie basse du doigt de massage 21 correspondant tandis que la partie haute dudit doigt de massage 21 porte la tête de travail 22. Les moyens de manœuvre comprennent alors une came 27 mobile en rotation sur elle-même selon une direction perpendiculaire à l'axe d'articulation 26 et coaxiale à l'arbre de sortie 8. La came 27 présente alors, au niveau d'une face inférieure, un logement de réception de l'arbre de sortie 8. La came 27 comprend, en outre, un chemin de came périphérique 28 dont le développé possède une forme sensiblement sinusoïdale. Chaque doigt de massage porte, à distance de l'axe d'articulation 26, un ergot 29 engagé dans le chemin de came 28 de sorte que la rotation de la came 27 sur elle-même induit un mouvement alternatif de chaque ergot 29. Compte tenu de la conformation des doigts de massage 21 le mouvement alternatif de chaque ergot 29 induit une oscillation alternative des doigts de massage en rotation autour de l'axe d'articulation 26.

De manière préférée, l'appareil de massage A et, plus particulièrement, les moyens de manœuvre 25, les moyens d'entraînement 7, et l'unité de commande 10 sont adaptés pour assurer à chacun des doigts de massage une fréquence de déplacement supérieure ou égale à 2,5Hz.

Il doit être remarqué que, selon l'exemple illustré, les moyens de manœuvre 25 sont adaptés pour coordonner le déplacement des doigts de massage 21 de manière que lorsque l'un des doigts de massage 21 est en position d'extension E, l'autre doigt de massage 21 est en position de rétraction R et réciproquement.

L'appareil de massage ainsi constitué est mis en œuvre de la manière suivante. La surface d'appui S est placée contre le visage puis l'utilisateur met en marche l'appareil de massage A au moyen de l'interface 11, les doigts de massage 21 s'animent alors d'un mouvement d'oscillation qui reproduit le geste de massage qui serait effectué au moyen de deux doigts par exemple l'index et le majeur venant tapoter alternativement la peau du visage notamment celle se situant autour des yeux.

Selon l'exemple illustré et afin de simuler au mieux le geste des doigts, chaque tête de travail comprend une surface de travail T de forme convexe et dans le cas présent de forme sphérique et rigide.

Le massage réalisé au moyen de l'appareil selon l'invention procure un effet stimulant, un effet lissant, un effet relaxant et permet notamment de réduire les cernes ou les poches sous les yeux, et de combler les rides et ridules. Afin d'optimiser ce traitement, l'appareil de massage A tel qu'illustré aux figures 38 à 40 comprend des moyens 40 d'application ou de distribution de produit cosmétique. Selon l'exemple illustré, les moyens d'application de produits cosmétique 40 comprennent un réservoir 41 situé dans le boitier d'entraînement 2 et raccordé, via un système de prélèvement 42, par exemple une pompe de prélèvement 42, à une buse de distribution 43 située dans l'élément d'appui 20. La pompe de prélèvement est pilotée par l'unité de commande 10 de manière à assurer la distribution de produit cosmétique lors du fonctionnement de l'appareil de massage A. Bien entendu, l'une des têtes de travail, voire les deux têtes de travail, pourrait comprendre une buse de distribution de produit cosmétique.

Par ailleurs, toujours selon l'exemple illustré aux figures 38 à 40, l'appareil de massage A comprend aussi des moyens d'application d'un courant électrique sur la peau 45 qui comprennent une unité 46 de génération d'un courant et/ou d'une tension électriques. L'unité de génération 46 est pilotée par l'unité de commande 10. L'unité de génération 46 est raccordée à une électrode 47 portée par une tête de travail 22.

Lors de l'utilisation de l'appareil de massage A, l'unité de commande 10 pilote le fonctionnement de l'unité de génération 46 de sorte que lorsque l'électrode 47 est en contact avec la peau un phénomène d'électrophorèse est induit qui favorise l'assimilation des principes actifs du produit cosmétique.

Selon l'invention, les doigts de massage 21 ne sont pas nécessairement animés d'un mouvement d'oscillation d'autour d'un axe horizontal. Ainsi les figures 41 et 42 montrent une tête de massage 1 pour un appareil selon l'invention dont les doigts de massage 21 possèdent une cinématique différente.

Selon cet exemple, chaque doigt de massage 21 est réalisé sous la forme d'une sorte de piston rectiligne qui s'étend en partie au moins à l'extérieur d'un corps creux 50 entourant les moyens de manœuvre 25. Chaque doigt est alors guidé en translation par un alésage 51 aménagé dans le corps creux 50. L'extrémité de chaque doigt de massage 21 située à l'intérieur du corps creux 50 coopère avec un pion excentré 52 porté par un disque de manœuvre 53 appartenant aux moyens de manœuvre 25. Le pion excentré 52 est disposé dans une chambre 54 qui est liée rigidement au doigt de massage correspondant et dans laquelle le pion excentré 52 peut se déplacer en translation de sorte que sa rotation avec le disque de manœuvre 53 est transformée en une translation du doigt de massage 21 correspondant.

De plus, selon cet exemple de réalisation, la surface d'appui S est plane.

Toujours selon cet exemple de réalisation, le débattement ou la course des doigts de massage n'est pas perpendiculaire à l'axe longitudinal L du boitier d'entrainement, mais forme un angle non nul avec ce dernier, selon l'exemple illustré un angle d'environ 30°. Ce mode de réalisation présente l'avantage de permettre à l'utilisateur d'atteindre plus facilement les zones étroites du visage comme le contour des yeux ou le contour de la bouche, sans être gêné par le corps de l'appareil.

Par ailleurs, selon cet exemple de réalisation, l'élément d'appui 20 porte des électrodes 56 de transmission d'un courant électrique à la peau.

Toujours selon cet exemple de réalisation, la tête de massage comprend des moyens 60 de diffusion de lumière en direction du visage. Dans le cas présent les moyens de diffusion 60 sont aménagés dans un doigt de massage 21 et comprennent une source de lumière 61 telle qu'une diode électroluminescente pilotée par l'unité de commande 10. La source de lumière 61 est alors associée à un système optique 62 comprenant une face de sortie D située au niveau de la surface de travail T et donc destinée à être orientée vers le visage de l'utilisateur de l'appareil de massage A selon l'invention.

La figure 43 illustre encore une autre variante de réalisation d'une tête de massage 1 d'un appareil de massage A conforme à l'invention. Selon cette variante, les deux doigts de massage 21 sont portés par une même platine 65 qui oscille en rotation autour d'un axe vertical en étant entrainé, via un alésage présent sous la platine, par un disque à excentrique 66 appartenant aux moyens de manœuvre 25 et dont le pion est disposé dans l'alésage de la platine. Selon cet exemple de réalisation, chaque doigt de massage 21 porte un capuchon amovible 67 qui forme la tête de travail 22 correspondante.

Selon un autre exemple de réalisation, ce capuchon peut être en matériau souple, type élastomère.

Selon un autre mode de réalisation, il peut comprendre un tampon 68 imbibé de produit cosmétique formant la surface de travail T. Les capuchons amovibles 67 forment ainsi des moyens d'application ou de distribution de produit cosmétique.

Selon cet exemple de réalisation, l'élément d'appui 20 comprend un système optique 62 de diffusion de la lumière produit par une source située soit dans la tête de massage 1 soit dans le boitier d'entrainement 2.

La figure 44 illustre une variante de réalisation de la tête de massage illustrée aux figures 41 et 42 dont elle partage la cinématique de manœuvre des doigts de massage 21. Cette variante de réalisation diffère de la tête de massage illustrée figures 41 et 42 en ce que les doigts de massage se déplacent en translation alternative selon une direction perpendiculaire à l'axe longitudinal L du boitier d'entrainement. De plus, selon cette forme de réalisation, la surface de travail T de chaque doigt de massage est plane.

### En ce qui concerne la tête appelée tête de zone de travail intérieure, illustrée en figures 45 à 49 avec ses propres références numériques :

Il est à noter que sur ces figures les éléments structurels et/ou fonctionnels communs aux différentes variantes peuvent présenter les mêmes références.

Un appareil de massage selon l'invention, tel qu'illustré à la figure 45 et désigné dans son ensemble par la référence A, comprend une tête de massage 1 adaptée de manière amovible sur un boîtier d'entraînement 2. La tête de massage 1 est conçue pour exercer une action mécanique sur la peau du visage d'un utilisateur par l'intermédiaire d'éléments de massage entraînés par un moteur électrique.

À cet effet, le boîtier d'entraînement 2 comprend un corps allongé de forme générale cylindrique qui comprend au niveau d'une de ses extrémités des moyens d'adaptation 5 de manière amovible de la tête de massage 1. Les moyens d'adaptation 5 sont, selon l'exemple illustré, formés par un fourreau à l'intérieur duquel la tête de massage 1 est en partie engagée.

Le boîtier d'entraînement 2 comprend, à l'intérieur du corps 3, un moteur électrique 6 qui actionne des moyens d'entraînement 7 adaptés pour transmettre le mouvement du moteur électrique aux éléments de massage de la tête de massage 1. Selon l'exemple illustré, les moyens d'entraînement 7 comprennent un réducteur non représenté qui entraîne un arbre de sortie 8 accessible au niveau des moyens d'adaptation 5 du boîtier d'entraînement 2.

Le moteur électrique 6 est piloté par une unité de commande 10 alimentée par un bloc de batterie 11 disposé à l'intérieur du corps. Bien entendu, l'alimentation électrique de l'unité de commande 10 pourrait également être effectuée directement à partir du réseau électrique par l'intermédiaire d'un transformateur. L'unité de commande 10 est en outre raccordée à une interface de commande manuelle 14 accessible depuis l'extérieur du corps. L'interface de commande manuelle 14 peut par exemple comprendre un interrupteur marche-arrêt et/ou des moyens de sélection manuelle de programmes de fonctionnement.

Le boîtier d'entrainement 2 comprend aussi des moyens de reconnaissance 12 qui sont raccordés à l'unité de commande 10 et qui sont adaptés pour lire des moyens d'identification 13 portés par la tête de massage 1. L'unité de commande 10 est alors adaptée pour contrôler le fonctionnement de l'appareil de massage A en fonction de la tête de massage 1 telle que reconnue suite à la lecture des moyens d'identification 13. Le contrôle du fonctionnement de l'appareil de massage A peut notamment consister en une détermination de la vitesse de rotation du moteur électrique 6 de manière qu'elle soit adaptée au massage devant être réalisé par les éléments de massage. Les moyens d'identification 13 peuvent, par exemple, être constitués par une puce RFID tandis que les moyens de reconnaissance 12 seront adaptés pour lire une telle puce RFID. Bien entendu, les moyens d'identification 13 et de reconnaissance 12 pourraient être réalisés de toute autre manière appropriée comme par exemple sous la forme d'un système d'identification par contact mécanique ou électrique, ou encore sous la forme d'un système d'identification magnétique mettant en œuvre des aimants permanents et des interrupteurs à lame souple (ILS).

Selon l'invention, la tête de massage 1 est conçue pour réaliser un massage par déplacement d'une tête de travail contre la peau, la tête restant en contact permanent avec la peau. À cet effet, la tête de massage 1 comprend, comme le montre la figure 46 un élément d'appui 20 qui est destiné à venir contre le visage. L'élément d'appui 20 présente une forme générale annulaire et forme une couronne d'appui 21 qui définit une surface d'appui S s'inscrivant dans un plan d'appui P, ladite surface d'appui S étant conçue pour pouvoir épouser entièrement la peau si l'utilisateur le souhaite. Selon l'exemple illustré, l'élément d'appui 20 est creux et rigide tandis que la surface d'appui S est formée par une matière élastiquement déformable telle qu'un élastomère (par exemple en silicone ou en EPDM). Ainsi, la surface d'appui S peut se déformer de quelques millimètres, de préférence moins de deux, afin d'épouser la forme de la région du visage contre laquelle elle est appliquée.

La couronne d'appui 21 délimite une zone de travail Z à l'intérieur de laquelle se trouvent au moins, selon l'exemple illustré, trois têtes de travail 22 qui possèdent chacune une surface de travail T s'étendant en saillie du plan d'appui P. Selon l'exemple illustré, chaque tête de travail 22 comprend une bille 23 de sorte de la surface de travail T correspondante possède une forme convexe et plus particulièrement sphérique. Chaque surface de travail T s'étend alors, de manière permanente, en saillie du plan d'appui P d'une hauteur H, mesurée entre le sommet de la surface de travail T et le plan d'appui P, de préférence égale ou supérieure au rayon de courbure R de ladite bille. Selon une variante, La hauteur H de saillie de chaque surface de travail T est supérieure à la moitié du rayon de courbure R mais inférieur au rayon de courbure R.

Selon l'exemple illustré, chaque bille 23 appartenant à une tête de travail 22 est réalisée en une matière rigide telle que du métal. Ainsi, chaque surface de travail T est rigide par opposition au caractère souple ou élastiquement déformable de la surface d'appui S. De plus, selon cet exemple la surface de chaque bille 23 est lisse.

Selon l'invention, la tête de massage 1 comprend également des moyens de manœuvre 25 adaptés pour déplacer chaque tête de travail 22 en rotation selon un axe Δ décalé par rapport au centre de chaque surface de travail T correspondante. Les moyens de manœuvre 25 sont alors adaptés pour coopérer avec les moyens d'entraînement 7 et plus particulièrement avec l'arbre de sortie 8 de manière à transmettre et transformer le mouvement de rotation du moteur électrique 6 en un mouvement de rotation des têtes de travail 22.

Selon l'exemple illustré et comme cela ressort des figures 46 et 47, les moyens de manœuvre 25 sont adaptés pour assurer un mouvement planétaire aux trois billes 23, c'est-à-dire un mouvement de rotation principal des trois billes 23 autour de l'axe de rotation Δ associé à un mouvement de rotation secondaire de chacune des billes autour d'un axe de rotation secondaire Δ' qui se déplace en rotation autour de l'axe de rotation Δ lors du fonctionnement de l'appareil de massage selon l'invention.

À cet effet, chaque tête de travail 22 comprend un disque 26 qui est centré par rapport à l'axe de rotation secondaire Δ' et qui porte sur sa face supérieure la bille 23 correspondante liée de manière rigide audit disque 26. Chaque tête de travail comprend en outre un pignon planétaire 27 qui est lié de manière rigide à la face inférieure du disque 26 correspondant et qui est coaxial à l'axe de rotation secondaire Δ'.

Les moyens de manœuvre 25 comprennent alors un arbre de manœuvre 28 d'axe Δ qui comprend sur une face intérieure un logement 29 de réception de l'arbre de sortie 8 et qui porte sur une face supérieure trois pions 24 engagés chacun dans un alésage axial d'un pignon planétaire 27. Les moyens de manœuvre comprennent en outre une couronne périphérique fixe 30 liée de manière rigide à un carter 31 constitutif de la tête de massage 1. Chacun des pignons planétaires 27 engrène la couronne périphérique fixe 30 de sorte que la rotation de l'arbre de manœuvre 28 entraîne le mouvement planétaire des billes 23 décrit précédemment.

Dans cette variante illustrée, on peut inverser les matières utilisées pour les billes et pour la couronne d'appui, c'est-à-dire, billes en matière souple et la couronne en matière rigide.

En fonction du besoin et dans le but d'atténuer le pétrissage, on peut aussi construire la tête de massage 1 de façon à ce que chaque tête de travail 22 soit montée sur l'axe Δ' et à ce qu'elle exerce une rotation régulière sur elle-même.

L'appareil de massage ainsi constitué est mis en œuvre de la manière suivante. La surface d'appui S est placée contre le visage puis l'utilisateur met en marche l'appareil de massage A au moyen de l'interface de commande manuel 14, les têtes de travail 22 s'animent alors d'un mouvement de rotation planétaire alors que la peau autour de la zone de travail Z est maintenue par la surface d'appui S de forme annulaire.

Le massage réalisé au moyen de l'appareil selon l'invention permet notamment de pétrir la peau du visage en profondeur et de réduire ainsi les rides en stimulant la circulation sanguine et en relançant la production des éléments constitutifs de la peau.

Afin d'optimiser ce traitement, l'appareil de massage A tel qu'illustré à la figure 45 comprend des moyens 40 d'application de produit cosmétique. Selon l'exemple illustré, les moyens d'application de produit cosmétique 40 comprennent un réservoir 41 situé dans le boitier d'entraînement 2 et raccordé, via un système de prélèvement 42, par exemple une pompe, à une buse de distribution 43 située dans l'élément d'appui 20. La pompe de prélèvement 42 est pilotée par l'unité de commande 10 de manière à assurer la distribution de produit cosmétique lors du fonctionnement de l'appareil de massage A. Bien entendu, l'élément d'appui 20 pourrait comprendre plus d'une buse de distribution. De plus, chaque tête de travail pourrait également comprendre une buse de distribution de produit cosmétique qui serait alimentée à partir d'un réservoir souple aménagé dans la tête de travail correspondante et sollicité par un système de came lors de la rotation de ladite tête de travail.

Par ailleurs, toujours selon l'exemple illustré aux figures 45 à 47, l'appareil de massage A comprend aussi des moyens d'application d'un courant électrique 45 qui comprennent une unité 46 de génération d'un courant et/ou d'une tension électriques. L'unité de génération 46 est pilotée par l'unité de commande 10. L'unité de génération 46 est raccordée à une électrode 47 portée par l'élément d'appui 20.

Lors de l'utilisation de l'appareil de massage A, l'unité de commande 10 pilote le fonctionnement de l'unité de génération 46 de sorte que lorsque l'électrode 47 est en contact avec la peau un phénomène d'électrophorèse est induit qui favorise l'assimilation des principes actifs du produit cosmétique.

Selon l'invention, les têtes de travail 22 ne sont pas nécessairement animées d'un mouvement de type planétaire tel que décrit précédemment.

Ainsi, la figure 48 illustre une variante de réalisation d'une tête de massage pour un appareil de massage selon l'invention qui diffère de celle décrite en relation avec les figures 45 à 47, en ce que les trois têtes de travail 22 sont portées par un plateau circulaire 50 lié en rotation à l'arbre de manœuvre 28. Le plateau 50 porte les trois têtes de travail 22 formées par les billes de métal 23 dont les centres sont décalés par rapport à l'axe de rotation Δ. Par ailleurs, les billes 23 sont chacune enfermée dans une coupelle hémisphérique 51 liée rigidement au plateau 50. Ainsi, chaque bille 23 est mobile en rotation sur elle-même de manière qu'elle peut rouler sur la peau lors de l'utilisation de l'appareil de massage selon cette variante de réalisation.

De plus, selon cet exemple de réalisation, chaque tête de travail comprend une électrode d'électrostimulation formée par la bille 23 correspondante qui est raccordée électriquement à l'unité de génération d'un courant 46 via un système de balais 52, 53 et d'une piste annulaire 54.

Toujours selon cet exemple de réalisation, la tête de massage comprend des moyens 60 de diffusion de lumière en direction du visage. Dans le cas présent les moyens de diffusion 60 sont aménagés dans la tête de massage et comprennent en tant que source de lumière, des diodes électroluminescentes 61 pilotées par l'unité de commande 10. Les sources de lumière 61 sont alors associées à un système optique formé par l'élément d'appui qui est transparent et qui comprend une face de sortie de la lumière située au niveau de la surface d'appui S et donc destinée à être orientée vers le visage de l'utilisateur de l'appareil de massage A selon l'invention.

Pour ce faire, toute la zone de travail (Z) peut être constituée de pièces transparentes afin de pouvoir faire passer la lumière.

La figure 48 illustre encore une autre variante de réalisation d'une tête de massage 1 d'un appareil de massage A conforme à l'invention. Selon cette variante, la tête de massage 1 ne comprend qu'une seule tête de travail 22 de forme sphérique qui est liée de manière rigide au plateau 50 en étant décalée par rapport à son centre. Selon cet exemple de réalisation, le centre de la tête de travail 22 est décalé par rapport au centre de la zone de travail Z par lequel passe l'axe Δ d'une distance d supérieure ou égale à 1/6 de la plus petite dimension de la zone de travail Z, ici le diamètre de la zone de travail Z, et inférieure à la moitié de ladite plus petite dimension de la zone de travail Z.

Selon cet exemple de réalisation, la tête de travail 22 porte un capuchon amovible 65 qui forme une surface de travail correspondante et comprend au niveau de son sommet un tampon 66 imbibé de produit cosmétique. Le capuchon amovible 65 forme ainsi un moyen d'application de produit cosmétique.

Selon cet exemple de réalisation, le capuchon 65 comprend en outre un système optique 67 de diffusion de la lumière produit par les sources de lumière 61 situées dans la tête de travail 22. Le système optique 67 est ici formé par une calotte sphérique en matériau transparent qui assure une fonction de guide de lumière.

### En ce qui concerne la tête appelée tête de pincé Jacquet, illustrée en figures 50 à 56 avec ses propres références numériques :

Il est à noter que sur ces figures les éléments structurels et/ou fonctionnels communs aux différentes variantes peuvent présenter les mêmes références.

Un appareil de massage selon l'invention, tel qu'illustré à la figure 50 et désigné dans son ensemble par la référence A, comprend une tête de massage 1 adaptée de manière amovible sur un boîtier d'entraînement 2. La tête de massage 1 est conçue pour exercer une action mécanique sur la peau du visage d'un utilisateur par l'intermédiaire d'éléments de massage E entraînés par un moteur électrique.

À cet effet, le boîtier d'entraînement 2 comprend un corps allongé 3 de forme générale cylindrique qui comprend au niveau d'une de ses extrémités 4 des moyens 5 d'adaptation de manière amovible de la tête de massage 1. Les moyens d'adaptation 5 sont, selon l'exemple illustré, formés par un fourreau à l'intérieur duquel la tête de massage 1 est en partie engagée.

Le boîtier d'entraînement 2 comprend, à l'intérieur du corps 3, un moteur électrique 6 qui actionne des moyens d'entraînement 7 adaptés pour transmettre le mouvement du moteur électrique aux éléments de massage de la tête de massage 1. Selon l'exemple illustré, les moyens d'entraînement 7 comprennent un réducteur non représenté qui entraîne un arbre de sortie 8 accessible au niveau des moyens d'adaptation 5 du boîtier d'entraînement 2.

Le moteur électrique 6 est piloté par une unité de commande 10 alimentée par un bloc de batterie B disposé à l'intérieur du corps 3. Bien entendu, l'alimentation électrique de l'unité de commande 10 pourrait également être effectuée directement à partir du réseau électrique par l'intermédiaire d'un transformateur. L'unité de commande 10 est en outre raccordée à une interface de commande manuelle 11 accessible depuis l'extérieur du corps 3. L'interface de commande manuelle 11 peut par exemple comprendre un interrupteur marche-arrêt et/ou des moyens de sélection manuelle de programmes de fonctionnement.

Le boîtier d'entrainement 2 comprend aussi des moyens de reconnaissance 12 qui sont raccordés à l'unité de commande 10 et qui sont adaptés pour lire des moyens d'identification 13 portés par la tête de massage 1. L'unité de commande 10 est alors adaptée pour contrôler le fonctionnement de l'appareil de massage A en fonction de la tête de massage 1 telle que reconnue suite à la lecture des moyens d'identification 13. Le contrôle du fonctionnement de l'appareil de massage A peut notamment consister en une détermination de la vitesse de rotation du moteur électrique 6 de manière qu'elle soit adaptée au massage devant être réalisé par les éléments de massage E. Les moyens d'identification 13 peuvent, par exemple, être constitués par une puce RFID tandis que les moyens de reconnaissance 12 seront adaptés pour lire une telle puce RFID. Bien entendu, les moyens d'identification 13 et de reconnaissance 12 pourraient être réalisés de toute autre manière appropriée comme par exemple sous la forme d'un système d'identification par contact mécanique ou électrique ou encore sous la forme d'un système d'identification magnétique mettant en œuvre des aimants permanents et des interrupteurs à lame souple (ILS) également appelés interrupteurs Reed.

Selon l'invention, la tête de massage 1 est conçue pour réaliser un massage par pincement. À cet effet, la tête de massage 1 comprend, en tant qu'éléments de massage E, deux rouleaux de massage 21 et 22 qui sont mobiles en rotation sur eux-mêmes selon deux axes de rotation Δ et Δ' parallèles entre eux et à une surface d'application S mieux visible à la figure 51. Les deux rouleaux de massage 21 et 22 sont distants l'un de l'autre en étant séparés par une zone de travail Z. Selon l'exemple illustré, la distance entre les axes de rotation Δ et Δ' est constante.

Par ailleurs, selon l'exemple illustré, les rouleaux de massage 21 et 22 sont agencés au sein de la tête de massage 1 de manière qu'un plan P tangent aux deux rouleaux et situé vers l'extérieur de la tête de massage forme avec un axe longitudinal du boîtier d'entraînement 2 un angle α non nul et différent de l'angle droit.

Conformément à l'invention, un premier rouleau 21 comprend au moins une palette, et dans le cas présent représenté à titre d'exemple quatre palettes, 23 à 27 qui s'étendent radialement en saillie de la surface du premier rouleau 21. La surface périphérique du premier rouleau 21 est située en retrait de la surface d'application S. Les palettes 23 à 27 présentent une extension radiale suffisante pour se trouver en saillie de la surface d'application S au fur et à mesure de la rotation du premier rouleau 21. Les palettes 23 à 27 sont en outre réparties régulièrement à la périphérie du premier rouleau 21 et sont, dans le cas présent, placées à 90° les unes des autres.

Selon l'exemple illustré, les palettes 23 à 27 possèdent des sections droites transversales, visibles en figures 50 et 56, de formes différentes et étant entendu que toutes les palettes pourraient présenter une même forme. Ainsi, la palette 23 possède, vue en section droite transversale, une extrémité libre plus épaisse que le reste de la palette 23. La palette 24 présente quant à elle, vue en section droite transversale, une forme en « 8 ». La palette 25 possède, elle, vue en section droite transversale une forme droite. Enfin, la palette 26 possède, en section droite transversale, une forme allongée et effilée dont l'épaisseur se réduit en allant vers l'extrémité. Pour mieux comprendre, cette forme peut prendre la forme connue universellement de virgule dont par exemple la concavité au repos est orientée à l'opposé du sens de rotation du premier rouleau 21 comme cela apparaîtra par la suite. Une autre forme de palette 27 est illustrée en figure 56 et présente, en section droite transversale, une forme de L majuscule dont la base est à l'extrémité libre de la palette. Chacune de ces formes permet un massage d'intensité et d'effet différents.

Le deuxième rouleau 22 présente une surface périphérique de type lisse qui s'étend en saillie de la surface d'application S ou qui s'étend à flanc de la surface d'application (S).

On a défini la surface de type lisse plus haut. Comme illustré en figures 50 à 54, la surface de type lisse du rouleau 22 peut être absolument lisse. La figure 55 illustre un mode alternatif de rouleau à surface de type lisse : la surface est cannelée parallèlement à la hauteur du rouleau, de façon régulière. Ainsi lorsque le rouleau est en contact avec la peau, il définit un angle d'appui Sa dont on peut considérer qu'au moins environ la moitié de la surface extérieure comprise dans l'angle Sa d'appui du rouleau vers la peau est en contact avec la peau.

Selon l'invention, la tête de massage 1 comprend également des moyens de transmission 30 adaptés pour entraîner les rouleaux 21 et 22 de manière simultanée dans le même sens, allant de l'extérieur de la zone de travail vers l'intérieur de la zone de travail pour le premier rouleau, et de l'intérieur vers l'extérieur de la zone de travail pour le second rouleau, vu depuis l'extérieur de la tête de massage et tel qu'indiqué par les flèches F1 et F2. Les moyens de transmission 30 sont alors adaptés pour coopérer avec les moyens d'entraînement 7 et plus particulièrement avec l'arbre de sortie 8 de manière à transmettre et transformer le mouvement de rotation du moteur électrique selon l'axe longitudinal L en des mouvements de rotation selon les axes Δ et Δ' qui présentent une direction orthogonale à celle de l'axe longitudinal L.

Selon l'exemple illustré et comme cela ressort de la figure 52, les moyens de transmission 30 comprennent un train d'engrenage comprenant, tout d'abord, deux pignons tronconiques assurant un renvoi d'angle et, ensuite, des pignons droits assurant l'entraînement ensemble, à vitesse différente des rouleaux de massage 21 et 22.

Les moyens de transmission 30 sont de préférence adaptés pour assurer une vitesse de rotation du premier rouleau 21 supérieure à celle du deuxième rouleau 22 et dans le cas présent double de celle du deuxième rouleau 22.

L'appareil de massage ainsi constitué est mis en œuvre de la manière suivante. La surface d'application S est placée contre le visage puis l'utilisateur met en marche l'appareil de massage A au moyen de l'interface 11, les rouleaux de massage 21 et 22 s'animent alors de mouvements de rotation dans le même sens. La rotation du second rouleau 22 induit le déplacement de la tête de massage sur la peau tandis que les palettes du premier rouleau 21 reproduisent l'effet de pincement de la peau, l'espacement entre les palettes permettant de relâcher la peau successivement et au fur et à mesure de l'avancement de la tête de massage.

Le massage réalisé au moyen de l'appareil selon l'invention permet notamment de réduire les rides en stimulant la circulation sanguine dans la peau du visage ou encore dans la peau du cou. Afin d'optimiser ce traitement, l'appareil de massage A tel qu'illustré aux figures 50 à 52 comprend des moyens 40 d'application de produit cosmétique. Selon l'exemple illustré, les moyens d'application de produit cosmétique 40 comprennent un réservoir 41 situé dans le boitier d'entraînement 2 et raccordé, via une pompe de prélèvement 42, à deux buses de distribution 43 situées dans la surface d'application S et plus particulièrement à proximité immédiate du deuxième rouleau de massage 22 comme le montre la figure 51. Ainsi, le produit cosmétique est appliqué sur la peau par le deuxième rouleau de massage. La pompe de prélèvement 42 est pilotée par l'unité de commande 10 de manière à assurer la distribution de produit cosmétique lors du fonctionnement de l'appareil de massage A.

Bien entendu, la surface application S pourrait ne comprendre qu'une seule buse de distribution ou encore plusieurs buses de distribution disposées différemment. De même, la buse ou les buses de distribution de produit cosmétique pourraient être disposées à l'intérieur de la tête de massage 1 de manière à appliquer le produit cosmétique sur l'un ou l'autre voire les deux rouleaux de massage.

Par ailleurs, toujours selon l'exemple illustré aux figures 50 à 52, l'appareil de massage A comprend aussi des moyens d'application de courant électrique 45 qui comprennent une unité 46 de génération d'un courant et/ou d'une tension électrique. L'unité de génération 46 est pilotée par l'unité de commande 10. L'unité de génération 46 est raccordée, via un système de balai 47 à une électrode 56 formée par au moins partiellement la surface conductrice du deuxième rouleau 22.

Lors de l'utilisation de l'appareil de massage A, l'unité de commande 10 pilote le fonctionnement de l'unité de génération 46 de sorte par exemple qu'un microcourant de stimulation de la peau ou des muscles du visage soit appliqué ou encore qu'un phénomène d'électrophorèse favorisant l'assimilation des principes actifs du produit cosmétique soit induit.

Selon l'exemple illustré aux figures 50 à 52, la tête de massage 1 comprend des moyens 50 de diffusion de lumière en direction du visage. Dans le cas présent les moyens de diffusion 50 sont aménagés dans la tête de massage et plus particulièrement la surface d'application S. Les moyens de diffusion 50 comprennent en tant que source de lumière, deux diodes électroluminescentes 51 pilotées par l'unité de commande 10 et disposées de part et d'autre de la zone de travail Z. Ces sources de lumière 51 sont alors chacune associées à un système optique 52 formé par bloc transparent qui forme un guide de lumière et qui comprend une face de sortie de la lumière située au niveau de la surface d'appui S et donc destinée à être orientée vers le visage de l'utilisateur de l'appareil de massage A selon l'invention.

Les figures 53 et 54 illustrent une variante de réalisation d'une tête de massage pour un appareil de massage selon l'invention qui diffère de celle décrite en relation avec les figures 50 à 52, en ce que la surface d'application S comprend deux électrodes 55 raccordées à l'unité de génération 46 de courant électrique.

Toujours selon cet exemple de réalisation, la tête de massage 1 comprend en outre, en tant que moyens de distribution de produit cosmétique, deux capuchons 60 adaptés de manière amovible sur ladite tête de massage 1. Chaque capuchon 60 comprend alors un tampon 62 imbibé de produit cosmétique.

Par ailleurs selon cette variante, les moyens de diffusion 50 sont aménagés dans la tête de massage et comprennent en tant que source de lumière, des diodes électroluminescentes 65 pilotées par l'unité de commande 10. Les diodes électroluminescentes 65 éclairent l'intérieur de la tête de massage 1 de sorte que la zone de massage forme une face de sortie de la lumière. A cet égard, la partie supérieure de la tête de massage pourrait aussi être transparente de sorte que la surface d'application S formerait une face de sortie de la lumière.

Bien entendu, diverses autres modifications ou variantes de l'appareil et de la tête de massage selon l'invention, peuvent être envisagées dans le cadre des revendications annexées.

Bien entendu le système de reconnaissance est envisagé sur chacune des têtes décrites ici.

En fonctionnement, plusieurs programmes « anti-âge, antirides, rajeunissement, remodelage » sont proposés via l'IHM comme suit. La séance peut consister en l'enchainement de différents massages combinés en simultané avec la lumière intense (orange - rouge) et chaude, chaque tête étant adaptée à une zone et pour une gestuelle particulière, de préférence avec 5 à 10 minutes par tête.

Alternativement la séance peut consister en l'enchainement de différents massages avant ou après l'application de lumière intense (orange - rouge) et chaude. Dans un premier exemple, la première phase peut être un programme « chaleur, préparation de peau ». Elle comporte une chauffe progressive, une mesure de la température en continu (via un capteur de température relié aux moyens de contrôle) et quand la valeur de température est atteinte (seuil entre 37°C et 45°C, de préférence 41°C), l'appareil indique via l'IHM de façon visuelle, lumineuse ou même sonore ou vibratoire à l'utilisateur qu'il peut démarrer le programme sur la peau : un timer peut être alors déclenché et un asservissement de la température peut être commandé pour maintenir la température cible. Ceci prépare la peau pour le massage, vasodilate les vaisseaux, réveille les fibroblastes. Dans un deuxième exemple, la dernière phase peut être un programme « chaleur ». Ceci favorise l'élimination et le drainage et apporte un bien être en fin de soin. Ceci améliore le confort et l'efficacité du soin. La chauffe peut être produite par des LED infrarouge, par les radiateurs de LED, par un ou des éléments chauffants plats (piste ILO), par des résistances avec des éléments mécaniques de conduction, par des pistes sérigraphiées.

Comme dit plus haut, la chaleur peut être aussi programmée simultanément à un massage mécanique motorisé du type massage anti-âge.

L'appareil reconnait la tête mise en place sur le corps, adapte les paramètres du moteur (tension pour vitesse et limitation de courant en cas de pincement) et de la lumière. Il indique quand il faut changer de tête (par exemple au bout de 6 minutes). La tête appelée « tapotement » est recommandée pour le contour des yeux (intensité lumineuse un peu plus faible) pour d'une part stimuler la microcirculation et ainsi réduire les poches/cernes, et d'autre part activer le métabolisme dans les sillons pour relancer la production des éléments de structure de la peau et combler les rides et ridules. La tête appelée « zone intérieure de travail » comportant trois boules de massage : est recommandée pour les zones larges pour pétrir en profondeur, stimuler les fibroblastes et la microcirculation. La tête appelée pincé Jacquet comportant les deux rouleaux dont l'un est à surface dite lisse et l'autre présente des palettes, est recommandée pour stimuler les sillons afin de combler les rides. La tête appelée « double rouleaux » avec deux rouleaux tournant de façon synchronisée et inversée est recommandée pour masser en profondeur pour remodeler les traits du visage. L'ajustement automatique des paramètres est commandé selon le logiciel enregistré dans le microcontrôleur.

Aussi l'application d'un produit cosmétique, à la main ou avec le système de distribution de produit embarquée est facultatif, peut être proposé avant ou après un massage. La distribution de cosmétique sera manuelle ou automatique selon la tête choisie, elle peut se faire pendant le massage pour une meilleure pénétration du cosmétique, elle peut se faire avant le massage pour une lubrification du contact, une meilleure glisse, moins de frottements et moins d'irritations; elle peut se faire après le massage pour une bonne hydratation et une bonne action anti-âge ; elle peut se faire avant ou en même temps que l'application de la lumière pour une activation par la lumière et/ou une diminution des pertes lumineuses grâce à une meilleure absorption de la lumière dans la peau (moins de réflexion à l'interface); elle peut se faire après la chauffe pour une meilleur pénétration des actifs dans la peau.

Pour chaque traitement, il peut aussi être proposé une vitesse ou une fréquence de massage progressive pendant le fonctionnement. Ceci permet à la peau de s'habituer en douceur et donne plus de confort, ensuite le massage s'intensifie pour plus d'efficacité une fois la peau « réveillée » Cette gestion est pilotée par le microcontrôleur et timer (oscillateur quartz).

## Revendications

1. Appareil de massage (1) de la peau comprenant :
- un corps (2) qui comporte des moyens de motorisation (3),
- au moins un type de tête de massage (4, 5, 6, 7, 8, ta, bi, ja) qui comprend au moins deux éléments de massage,
- un mécanisme de transmission qui permet l'activation des éléments de massage sous l'action des moyens de motorisation,
- des moyens d'assemblage (9) qui sont configurés pour assembler de manière amovible l'au moins un type de tête de massage avec le corps,
- des moyens de reconnaissance (146) du type de tête de massage assemblé sur le corps,
**caractérisé en ce que** des moyens de commande (161) dudit appareil de massage qui, en fonction de la reconnaissance du type de tête de massage, sont configurés pour agir sur les moyens de motorisation (3) de sorte à commander le mouvement d'au moins deux éléments de la tête de massage (4, 5, 6, 7, 8, ta, bi, ja), lesdits moyens de commande (161) étant configurés pour modifier l'actionnement des moyens de motorisation de sorte à adapter la vitesse d'activation des éléments de massage de la tête de massage,
l'appareil de massage (1) comprenant une tête de massage (4) qui comprend :
- une surface d'application (31) et,
- où les éléments de massage sont constitués de deux rouleaux de massage agencés selon deux axes longitudinaux parallèles, avec un écartement entre eux, et dépassant partiellement à l'extérieur de la surface d'application et,
- dans lequel un mécanisme de transmission est configuré pour entraîner en rotation de manière synchronisée et inversée, les deux rouleaux de sorte que, dans une vue en plan perpendiculaire aux deux axes, la partie du rouleau de massage située à gauche qui dépasse de la surface d'application, tourne dans le sens trigonométrique et, la partie du rouleau de massage située à droite qui dépasse de la surface d'application, tourne dans le sens horaire.

2. Appareil de massage (1) de la peau comprenant :
- un corps (2) qui comporte des moyens de motorisation (3),
- au moins un type de tête de massage (4, 5, 6, 7, 8, ta, bi, ja) qui comprend au moins deux éléments de massage,
- un mécanisme de transmission qui permet l'activation des éléments de massage sous l'action des moyens de motorisation,
- des moyens d'assemblage (9) qui sont configurés pour assembler de manière amovible l'au moins un type de tête de massage avec le corps,
- des moyens de reconnaissance (146) du type de tête de massage assemblé sur le corps,
**caractérisé en ce que** des moyens de commande (161) dudit appareil de massage qui, en fonction de la reconnaissance du type de tête de massage, sont configurés pour agir sur les moyens de motorisation (3) de sorte à commander le mouvement d'au moins deux éléments de la tête de massage (4, 5, 6, 7, 8, ta, bi, ja), lesdits moyens de commande (161) étant configurés pour modifier l'actionnement des moyens de motorisation de sorte à adapter la vitesse d'activation des éléments de massage de la tête de massage,
l'appareil de massage (1) comprenant une tête de massage (1) qui comprend :
- deux éléments de massage constitués de deux rouleaux de massage (21,22) parallèles qui sont mobiles en rotation sur eux-mêmes selon deux axes de rotation parallèles entre eux et à la surface d'application (S), distants l'un de l'autre en étant séparés par une zone de travail (Z), un premier rouleau comprenant au moins une palette (23, 24, 25, 26, 27) qui s'étend radialement en saillie de la surface du premier rouleau (21), et le deuxième rouleau (22) présentant une surface de type lisse,
- le mécanisme de transmission permettant l'entrainement des rouleaux en rotation et dans le même sens, le premier rouleau allant de l'extérieur de la zone de travail (Z) vers l'intérieur de la zone de travail (Z), et le deuxième rouleau allant de l'intérieur de la zone de travail vers l'extérieur de la zone de travail, vu depuis l'extérieur de la tête de massage (1).

3. Appareil de massage (1) de la peau comprenant :
- un corps (2) qui comporte des moyens de motorisation (3),
- au moins un type de tête de massage (4, 5, 6, 7, 8, ta, bi, ja) qui comprend au moins deux éléments de massage,
- un mécanisme de transmission qui permet l'activation des éléments de massage sous l'action des moyens de motorisation,
- des moyens d'assemblage (9) qui sont configurés pour assembler de manière amovible l'au moins un type de tête de massage avec le corps,
- des moyens de reconnaissance (146) du type de tête de massage assemblé sur le corps,
**caractérisé en ce que** des moyens de commande (161) dudit appareil de massage qui, en fonction de la reconnaissance du type de tête de massage, sont configurés pour agir sur les moyens de motorisation (3) de sorte à commander le mouvement d'au moins deux éléments de la tête de massage (4, 5, 6, 7, 8, ta, bi, ja), lesdits moyens de commande (161) étant configurés pour modifier l'actionnement des moyens de motorisation de sorte à adapter la vitesse d'activation des éléments de massage de la tête de massage,
l'appareil de massage (1) comprenant une tête de massage (1) qui comprend :
- un élément d'appui (20) destiné à venir contre le visage et qui définit une surface d'appui (S),
- au dessus de l'élément d'appui (20) au moins un élément de massage constitué d'un doigt de massage (21) chacun comprenant une tête de travail (22) destinée à venir au contact du visage et chacun étant mobile entre :
- une position de rétraction (R) dans laquelle la tête de travail (22) est située en deçà de la surface d'appui (S) vers l'intérieur de la tête de massage (1),
- une position d'extension (E) dans laquelle la tête de travail (22) est située au delà de la surface d'appui (S) vers l'extérieur de la tête de massage (1),
- des moyens de manoeuvre (25) de chaque doigt de massage (21) à lier au mécanisme de transmission et adaptés pour déplacer chacun des doigts de massage (21) entre ses positions d'extension (E) et de rétraction (R) de manière alternative.

4. Appareil de massage (1) de la peau comprenant :
- un corps (2) qui comporte des moyens de motorisation (3),
- au moins un type de tête de massage (4, 5, 6, 7, 8, ta, bi, ja) qui comprend au moins deux éléments de massage,
- un mécanisme de transmission qui permet l'activation des éléments de massage sous l'action des moyens de motorisation,
- des moyens d'assemblage (9) qui sont configurés pour assembler de manière amovible l'au moins un type de tête de massage avec le corps,
- des moyens de reconnaissance (146) du type de tête de massage assemblé sur le corps,
**caractérisé en ce que** des moyens de commande (161) dudit appareil de massage qui, en fonction de la reconnaissance du type de tête de massage, sont configurés pour agir sur les moyens de motorisation (3) de sorte à commander le mouvement d'au moins deux éléments de la tête de massage (4, 5, 6, 7, 8, ta, bi, ja), lesdits moyens de commande (161) étant configurés pour modifier l'actionnement des moyens de motorisation de sorte à adapter la vitesse d'activation des éléments de massage de la tête de massage,
l'appareil de massage (1) comprenant une tête de massage (1) qui comprend :
- un élément d'appui (20) qui est destiné à venir contre le visage et qui forme une couronne d'appui (21) définissant, d'une part, une surface d'appui (S) s'inscrivant dans un plan d'appui (P) et, d'autre part, une zone de travail (Z) située à l'intérieur de la couronne d'appui,
- à l'intérieur de la couronne d'appui et dans la zone de travail (Z), au moins un élément de massage constitué d'une tête de travail (22) qui possède une surface de travail s'étendant en saillie du plan d'appui (P),
- des moyens de manoeuvre (25) à lier au mécanisme de transmission et adaptés pour déplacer en rotation chaque tête de travail (22) selon au moins un axe de rotation (Δ, Δ') décalé par rapport au centre de la surface de travail (T) correspondante.

5. Appareil de massage (1) selon l'une des revendications 1 à 4 où les moyens de commande (161) dudit appareil de massage (1) sont configurés pour agir sur les moyens de motorisation (3) de sorte à commander de façon liée un mouvement desdits au moins deux éléments de massage (4, 5, 6, 7, 8, ta, bi, ja).

6. Appareil de massage (1) selon l'une des revendications 1 à 4 où les moyens de commande (161) dudit appareil de massage (1) sont configurés pour agir sur les moyens de motorisation (3) de sorte à actionner en rotation chacun desdits au moins deux éléments de massage (4, 5, 6, 7, 8, ta, bi, ja).

7. Appareil de massage (1) selon l'une des revendications précédentes où les éléments de massage sont choisis parmi l'ensemble constitué de : embout de massage, doigt de massage, rouleau de massage, tête de travail, bille de massage.

8. Appareil de massage (1) selon l'une des revendications précédentes où les moyens de commande (161) dudit appareil de massage (1) sont configurés pour agir sur les moyens de motorisation (3) de sorte à commander au moins un parmi les paramètres suivants desdits au moins deux éléments de massage : la vitesse de rotation, le sens de rotation, la fréquence d'oscillation.

9. Appareil de massage (1) selon l'une des revendications précédentes comprenant des moyens d'émission d'ondes (106) et dans lequel les moyens de commande (161) sont configurés pour agir sur le système d'émission d'ondes (106) en fonction du type de tête de massage (4, 5, 6, 7, 8, ta, bi, ja) assemblé sur le corps (2).

10. Appareil de massage (1) selon la revendication précédente où les moyens d'émission d'ondes (106) sont des moyens d'émission d'ondes lumineuses parmi au moins une des longueurs d'ondes suivantes : longueurs d'ondes visibles comprenant le rouge, l'orange, et longueur d'onde de l'infrarouge.

11. Appareil de massage (1) selon la revendication 3 et la revendication 9 dans lequel les moyens de commande (161) sont configurés, à la reconnaissance de la tête, pour agir sur le système d'émission d'ondes lumineuses visibles orange

12. Appareil de massage (1) selon la revendication 4 et la revendication 9, dans lequel les moyens de commande (161) sont configurés pour, à la reconnaissance de la tête, agir sur le système d'émission d'ondes lumineuses infrarouges

13. Appareil de massage (1) selon l'une des revendications 9 ou 10 dans lequel les moyens de commande (161) sont configurés pour agir sur le système d'émission d'ondes (106) et modifier la ou les longueurs d'ondes et / ou l'intensité et /ou la fréquence d'émission des ondes en fonction du type de tête de massage (4, 5, 6, 7, 8, ta, bi, ja) assemblée sur le corps (2).

14. Appareil de massage (1) selon l'une des revendications 9, 10 ou 13 comprenant des moyens d'émission d'ondes (106) agencés sur le corps (2) et des moyens de transfert (108) des ondes, du système d'émission vers la tête de massage (4, 5, 6, 7, 8, ta, bi, ja).

15. Appareil de massage (1) selon l'une des revendications 9, 10, 13 ou 14 où les moyens d'émission d'ondes (106) comprennent des diodes électroluminescentes.

16. Appareil de massage (1) selon l'une des revendications précédentes dans lequel les moyens de commande (161) sont configurés pour agir simultanément sur le système d'émission d'ondes (106) et sur les moyens de motorisation (3) de sorte à commander en mouvement au moins deux éléments de la tête de massage (4, 5, 6, 7, 8, ta, bi, ja), ceci en fonction de la reconnaissance du type de tête de massage pour établir au moins deux phases différentes de traitement de la peau durant le traitement.

17. Appareil de massage (1) selon l'une des revendications précédentes, dans lequel les moyens de reconnaissance (13, 146) sont des capteurs mécaniques, magnétiques ou optiques (151, 152, 164, 165, 169, 170) agencés sur l'au moins un type de tête de massage (147) et le corps (2) et, configurés pour transmettre des informations aux moyens de commande (161) en fonction du type de tête de massage assemblé sur le corps.

18. Appareil de massage (1) selon l'une des revendications précédentes comprenant un système de distribution de produit cosmétique (77) agencé sur le corps et des moyens de transfert (90) du produit, du corps (2) vers la tête de massage (4, 5, 6, 7, 8, ta, bi, ja).

19. Appareil de massage (1) selon la revendication précédente, dans lequel les moyens de commande (161) sont configurés pour agir sur le système de distribution de produit cosmétique et modifier au moins la distribution du produit cosmétique en fonction du type de tête de massage (4, 5, 6, 7, 8, ta, bi, ja) assemblé sur le corps (2).

20. Appareil de massage (1) selon l'une des revendications précédentes, comprenant un dispositif de traitement ionophorèse transcutanée qui est configuré pour transmettre sur la peau lors de l'application dudit appareil de massage, un courant permettant d'augmenter et/ou d'accélérer la pénétration d'un produit cosmétique.

## Patentansprüche

1. Massagegerät (1) für die Haut, umfassend:
- einen Körper (2), der Motorisierungsmittel (3) aufweist,
- mindestens einen Typ von Massagekopf (4, 5, 6, 7, 8, ta, bi, ja), der mindestens zwei Massageelemente umfasst,
- einen Übertragungsmechanismus, der die Aktivierung der Massageelemente unter der Einwirkung der Motorisierungsmittel ermöglicht,
- Montagemittel (9), die so konfiguriert sind, dass sie den mindestens einen Typ von Massagekopf lösbar an dem Körper montieren,
- Mittel zur Erkennung (146) des Typs des an dem Körper montierten Massagekopfes,
**dadurch gekennzeichnet, dass** Steuermittel (161) des Massagegeräts, die in Abhängigkeit von der Erkennung des Typs des Massagekopfs so konfiguriert sind, dass sie auf die Motorisierungsmittel (3) einwirken, um die Bewegung von mindestens zwei Elementen des Massagekopfs (4, 5, 6, 7, 8, ta, bi, ja) zu steuern, wobei die Steuermittel (161) so konfiguriert sind, dass sie die Betätigung der Motorisierungsmittel ändern, um die Aktivierungsgeschwindigkeit der Massageelemente des Massagekopfes anzupassen,
wobei das Massagegerät (1) einen Massagekopf (4) umfasst, der umfasst:
- eine Applikationsfläche (31), und,
- wobei die Massageelemente aus zwei Massagerollen bestehen, die entlang zweier paralleler Längsachsen mit einem Abstand voneinander angeordnet sind und teilweise aus der Applikationsfläche herausragen, und
- wobei ein Übertragungsmechanismus so konfiguriert ist, dass er die beiden Rollen synchron und in umgekehrter Richtung dreht, so dass in einer Draufsicht senkrecht zu den beiden Achsen der Teil der Massagerolle, der sich links befindet und von der Applikationsfläche herausragt, sich in dem trigonometrischen Sinn dreht und der Teil der Massagerolle, der sich rechts befindet und von der Applikationsfläche herausragt, sich im Uhrzeigersinn dreht.

2. Massagegerät (1) für die Haut, umfassend:
- einen Körper (2), der Motorisierungsmittel (3) aufweist,
- mindestens einen Typ von Massagekopf (4, 5, 6, 7, 8, ta, bi, ja), der mindestens zwei Massageelemente umfasst,
- einen Übertragungsmechanismus, der die Aktivierung der Massageelemente unter der Einwirkung der Motorisierungsmittel ermöglicht,
- Montagemittel (9), die so konfiguriert sind, dass sie den mindestens einen Typ von Massagekopf lösbar an dem Körper montieren,
- Mittel zur Erkennung (146) des Typs des an dem Körper montierten Massagekopfes,
**dadurch gekennzeichnet, dass** Steuermittel (161) des Massagegeräts, die in Abhängigkeit von der Erkennung des Typs des Massagekopfs so konfiguriert sind, dass sie auf die Motorisierungsmittel (3) einwirken, um die Bewegung von mindestens zwei Elementen des Massagekopfs (4, 5, 6, 7, 8, ta, bi, ja) zu steuern, wobei die Steuermittel (161) so konfiguriert sind, dass sie die Betätigung der Motorisierungsmittel ändern, um die Aktivierungsgeschwindigkeit der Massageelemente des Massagekopfes anzupassen,
wobei das Massagegerät (1) einen Massagekopf (1) umfasst, der umfasst:
- zwei Massageelemente, die aus zwei parallelen Massagerollen (21, 22) bestehen, die um zwei zueinander und zu der Applikationsfläche (S) parallele Drehachsen um sich selbst drehbar sind, voneinander beabstandet und durch eine Arbeitszone (Z) getrennt sind, wobei eine erste Rolle mindestens einen Flügel (23, 24, 25, 26, 27) umfasst, der sich von der Oberfläche der ersten Rolle (21) radial vorstehend erstreckt, und wobei die zweite Rolle (22) eine Oberfläche vom glatten Typ vorweist,
- den Übertragungsmechanismus, der es ermöglicht, die Rollen in Drehung und in dem gleichen Sinn anzutreiben, wobei die erste Rolle von dem Äußeren des Arbeitsbereichs (Z) zu dem Inneren des Arbeitsbereichs (Z) verläuft, und wobei die zweite Rolle von dem Inneren des Arbeitsbereichs zu dem Äußeren des Arbeitsbereichs verläuft, von dem Äußeren des Massagekopfes (1) gesehen.

3. Massagegerät (1) für die Haut, umfassend:
- einen Körper (2), der Motorisierungsmittel (3) aufweist,
- mindestens einen Typ von Massagekopf (4, 5, 6, 7, 8, ta, bi, ja), der mindestens zwei Massageelemente umfasst,
- einen Übertragungsmechanismus, der die Aktivierung der Massageelemente unter der Einwirkung der Motorisierungsmittel ermöglicht,
- Montagemittel (9), die so konfiguriert sind, dass sie den mindestens einen Typ von Massagekopf lösbar an dem Körper montieren,
- Mittel zur Erkennung (146) des Typs des an dem Körper montierten Massagekopfes,
**dadurch gekennzeichnet, dass** Steuermittel (161) des Massagegeräts, die in Abhängigkeit von der Erkennung des Typs des Massagekopfs so konfiguriert sind, dass sie auf die Motorisierungsmittel (3) einwirken, um die Bewegung von mindestens zwei Elementen des Massagekopfs (4, 5, 6, 7, 8, ta, bi, ja) zu steuern, wobei die Steuermittel (161) so konfiguriert sind, dass sie die Betätigung der Motorisierungsmittel ändern, um die Aktivierungsgeschwindigkeit der Massageelemente des Massagekopfes anzupassen,
wobei das Massagegerät (1) einen Massagekopf (1) umfasst, der umfasst:
- ein Stützelement (20), das dazu bestimmt ist, an dem Gesicht anzuliegen, und das eine Stützfläche (S) definiert,
- über dem Stützelement (20) mindestens ein Massageelement, das aus einem Massagefinger (21) besteht, die jeweils einen Arbeitskopf (22) aufweisen, der bestimmt ist, mit dem Gesicht in Kontakt zu kommen, und jeweils beweglich ist zwischen:
- einer eingefahrenen Position (R), in der sich der Arbeitskopf (22) unter der Auflagefläche (S) zu dem Inneren des Massagekopfes (1) hin befindet,
- einer ausgefahrenen Position (E), in der sich der Arbeitskopf (22) über der Auflagefläche (S) zu dem Äußeren des Massagekopfes (1) hin befindet,
- Mittel (25) zur Betätigung jedes Massagefingers (21), die mit dem Übertragungsmechanismus zu verbinden sind und geeignet sind, jeden der Massagefinger (21) abwechselnd zwischen seiner ausgefahrenen (E) und eingefahrenen Position (R) zu bewegen.

4. Massagegerät (1) für die Haut, umfassend:
- einen Körper (2), der Motorisierungsmittel (3) aufweist,
- mindestens einen Typ von Massagekopf (4, 5, 6, 7, 8, ta, bi, ja), der mindestens zwei Massageelemente umfasst,
- einen Übertragungsmechanismus, der die Aktivierung der Massageelemente unter der Einwirkung der Motorisierungsmittel ermöglicht,
- Montagemittel (9), die so konfiguriert sind, dass sie den mindestens einen Typ von Massagekopf lösbar an dem Körper montieren,
- Mittel zur Erkennung (146) des Typs des an dem Körper montierten Massagekopfes,
**dadurch gekennzeichnet, dass** Steuermittel (161) des Massagegeräts, die in Abhängigkeit von der Erkennung des Typs des Massagekopfs so konfiguriert sind, dass sie auf die Motorisierungsmittel (3) einwirken, um die Bewegung von mindestens zwei Elementen des Massagekopfs (4, 5, 6, 7, 8, ta, bi, ja) zu steuern, wobei die Steuermittel (161) so konfiguriert sind, dass sie die Betätigung der Motorisierungsmittel ändern, um die Aktivierungsgeschwindigkeit der Massageelemente des Massagekopfes anzupassen,
wobei das Massagegerät (1) einen Massagekopf (1) umfasst, der umfasst:
- ein Stützelement (20), das dazu bestimmt ist, an dem Gesicht anzuliegen, und das einen Stützring (21) bildet, einerseits eine Stützfläche (S), die in einer Stützebene (P) liegt, und andererseits eine Arbeitszone (Z) definiert, die sich im Inneren des Stützrings befindet,
- im Inneren des Stützrings und im Arbeitsbereich (Z) mindestens ein Massageelement, das aus einem Arbeitskopf (22) besteht, der eine sich aus der Stützebene (P) hervorstehend erstreckende Arbeitsfläche besitzt,
- Mittel zur Betätigung (25), die mit dem Übertragungsmechanismus zu verbinden sind und geeignet sind, jeden Arbeitskopf (22) um mindestens eine Drehachse (Δ, Δ') zu drehen, die in Bezug auf die Mitte der entsprechenden Arbeitsfläche (T) versetzt ist.

5. Massagegerät (1) nach einem der Ansprüche 1 bis 4, wobei die Steuermittel (161) des Massagegeräts (1) so konfiguriert sind, dass sie auf die Motorisierungsmittel (3) einwirken, um eine Bewegung der mindestens zwei Massageelemente (4, 5, 6, 7, 8, ta, bi, ja) in gekoppelter Weise zu steuern.

6. Massagegerät (1) nach einem der Ansprüche 1 bis 4, wobei die Steuermittel (161) des Massagegeräts (1) so konfiguriert sind, dass sie auf die Motorisierungsmittel (3) einwirken, um jedes der mindestens zwei Massageelemente (4, 5, 6, 7, 8, ta, bi, ja) in Drehung anzutreiben.

7. Massagegerät (1) nach einem der vorstehenden Ansprüche, wobei die Massageelemente aus der Menge ausgewählt sind, die besteht aus: Massagespitze, Massagefinger, Massagerolle, Arbeitskopf, Massagekugel.

8. Massagegerät (1) nach einem der vorstehenden Ansprüche, wobei die Steuermittel (161) des Massagegeräts (1) so konfiguriert sind, dass sie auf die Motorisierungsmittel (3) einwirken, um mindestens einen der folgenden Parameter der mindestens zwei Massageelemente zu steuern: die Drehgeschwindigkeit, die Drehrichtung, die Schwingungsfrequenz.

9. Massagegerät (1) nach einem der vorstehenden Ansprüche, umfassend Mittel zum Aussenden von Wellen (106), und wobei die Steuermittel (161) so konfiguriert sind, dass sie auf das System zum Aussenden von Wellen (106) in Abhängigkeit von dem Typ des an dem Körper (2) montierten Massagekopfes (4, 5, 6, 7, 8, ta, bi, ja) einwirken.

10. Massagegerät (1) nach dem vorstehenden Anspruch, wobei die Mittel zum Aussenden von Wellen (106) Mittel zum Aussenden von Lichtwellen mit mindestens einer der folgenden Wellenlängen sind: sichtbare Wellenlängen einschließlich der roten, orangen und infraroten Wellenlänge.

11. Massagegerät (1) nach Anspruch 3 und Anspruch 9, wobei die Steuermittel (161) so konfiguriert sind, dass sie bei Erkennen des Kopfes auf das System zum Aussenden von sichtbaren, orangen Lichtwellen einwirken.

12. Massagegerät (1) nach Anspruch 4 und Anspruch 9, wobei die Steuermittel (161) so konfiguriert sind, dass sie bei Erkennung des Kopfes auf das System zum Aussenden von infraroten Lichtwellen einwirken.

13. Massagegerät (1) nach einem der Ansprüche 9 oder 10, wobei die Steuermittel (161) so konfiguriert sind, dass sie auf das System zum Aussenden von Wellen (106) einwirken und die Wellenlänge(n) und/oder die Intensität und/oder die Frequenz zum Aussenden der Wellen in Abhängigkeit von dem Typ des an dem Körper (2) montierten Massagekopfes (4, 5, 6, 7, 8, ta, bi, ja) ändern.

14. Massagegerät (1) nach einem der Ansprüche 9, 10 oder 13, umfassend Mittel zum Aussenden von Wellen (106), die auf dem Körper (2) angeordnet sind, und Mittel zur Übertragung (108) von Wellen von dem System zum Aussenden zu dem Massagekopf (4, 5, 6, 7, 8, ta, bi, ja).

15. Massagegerät (1) nach einem der Ansprüche 9, 10, 13 oder 14, wobei die Mittel zum Aussenden von Wellen (106) Leuchtdioden umfassen.

16. Massagegerät (1) nach einem der vorstehenden Ansprüche, wobei die Steuermittel (161) so konfiguriert sind, dass sie gleichzeitig auf das System zum Aussenden von Wellen (106) und auf die Motorisierungsmittel (3) einwirken, um mindestens zwei Elemente des Massagekopfes (4, 5, 6, 7, 8, ta, bi, ja) in Bewegung zu steuern, dies als Funktion der Erkennung des Typs des Massagekopfs, um mindestens zwei verschiedene Phasen der Hautbehandlung während der Behandlung festzulegen.

17. Massagegerät (1) nach einem der vorstehenden Ansprüche, wobei die Erkennungsmittel (13, 146) mechanische, magnetische oder optische Sensoren (151, 152, 164, 165, 169, 170) sind, die auf dem mindestens einen Typ von Massagekopf (147) und dem Körper (2) angeordnet und so konfiguriert sind, dass sie Informationen an Steuermittel (161) in Abhängigkeit von dem Typ des auf dem Körper montierten Massagekopfes übertragen.

18. Massagegerät (1) nach einem der vorstehenden Ansprüche, umfassend ein an dem Körper angeordnetes Abgabesystem (77) für ein kosmetisches Produkt und Mittel (90) zur Übertragung des Produkts von dem Körper (2) zu dem Massagekopf (4, 5, 6, 7, 8, ta, bi, ja).

19. Massagegerät (1) nach dem vorstehenden Anspruch, wobei die Steuermittel (161) so konfiguriert sind, dass sie auf das System zur Ausgabe eines kosmetischen Produkts einwirken und zumindest die Ausgabe des kosmetischen Produkts in Abhängigkeit von dem Typ des auf dem Körper (2) montierten Massagekopfes (4, 5, 6, 7, 8, ta, bi, ja) ändern.

20. Massagegerät (1) nach einem der vorstehenden Ansprüche, umfassend eine Vorrichtung zur transkutanen Iontophorese-Behandlung, die so konfiguriert ist, dass sie bei Anwendung des Massagegeräts einen Strom, der die Erhöhung und/oder Beschleunigung der Penetration eines kosmetischen Produkts ermöglicht, auf die Haut überträgt.

## Claims

1. A massaging appliance (1) for the skin, comprising:
- a body (2) that includes a driving means (3),
- at least one type of massaging head (4, 5, 6, 7, 8, ta, bi, ja) that includes at least two massaging elements,
- a transmission mechanism that activates the massaging elements via the propulsion of the driving means,
- means of attachment (9) that are configured to attach, in a removable manner, the at least one type of massaging head to the body,
- means of distinguishing (146) the type of massaging head attached to the body
**characterized in that** control means (161) of said massaging appliance which, depending on the type of massaging head distinguished, are configured to act upon the driving means (3) so as to control the movement of at least two elements in the massaging head (4, 5, 6, 7, 8, ta, bi, ja), said control means (161) being configured to modify the activating of the driving means so as to adapt the activation speed of the massaging elements of the massaging head,
the massaging appliance (1) comprising a massaging head (4) that includes:
- an application surface (31) and,
- in which the massaging elements consist of two massaging rollers positioned along two parallel longitudinal axes with a gap between them, and partially extending beyond the exterior of the application surface, and
- in which a transmission mechanism is configured to spin the two rollers in an inverse synchronised manner, such that when viewed along a plane perpendicular to the two axes, the portion of the massaging roller located on the left, which extends beyond the application surface, spins in the trigonometric direction and the portion of the massaging roller located on the right, that extends beyond the application surface, spins clockwise.

2. A massaging appliance (1) for the skin comprising:
- a body (2) that includes driving means (3),
- at least one type of massaging head (4, 5, 6, 7, 8, ta, bi, ja) that includes at least two massaging elements,
- a transmission mechanism that activates the massaging elements via the propulsion of the driving means,
- means of attachment (9) that area configured to attach, in a removable manner, the at least one type of massaging head to the body,
- means of distinguishing (146) the type of massaging head attached to the body
**characterized in that** control means (161) of said massaging appliance which, depending on the type of massaging head distinguished, are configured to act upon the driving means (3) so as to control the movement of at least two elements in the massaging head (4, 5, 6, 7, 8, ta, bi, ja), said control means (161) being configured to modify the activating of the driving means so as to adapt the activation speed of the massaging elements of the massaging head,
the massaging appliance (1) comprising a massaging head (1) that includes:
- two massaging elements consisting of two parallel massaging rollers (21, 22) that can spin on themselves along two axes of rotation that are parallel to one another and to an application surface (S) separated from one another by a work zone (Z), a first roller having at least one paddle (23, 24, 25, 26, 27) that extends radially to protrude from the surface of the first roller (21), and wherein the second roller (22) has a smooth surface,
- the transmission mechanism allowing the spinning of the rollers in the same direction, the first roller going from the exterior of the work zone (Z) toward an interior of the work zone (Z), and the second roller going from the interior of the work zone toward the exterior of the work zone, as viewed from an exterior of the massaging head (1).

3. A massaging appliance (1) for the skin, comprising:
- a body (2) that includes driving means (3),
- at least one type of massaging head (4, 5, 6, 7, 8, ta, bi, ja) that includes at least two massaging elements,
- a transmission mechanism that activates the massaging elements via the propulsion of the driving means,
- means of attachment (9) that are configured to attach, in a removable manner, the at least one type of massaging head to the body,
- means of distinguishing (146) the type of massaging head attached to the body
**characterized in that** control means (161) of said massaging appliance which, depending on the type of massaging head distinguished, are configured to act upon the driving means (3) so as to control the movement of at least two elements of the massaging head (4, 5, 6, 7, 8, ta, bi, ja), said control means (161) being configured to modify the activating of the driving means so as to adapt the activation speed of the massaging elements of the massaging head,
the massaging appliance (1) comprising a massaging head (1) that includes:
- a pressure element (20) intended to be pressed against the face and defines a pressure surface (S),
- above the pressure element (20) at least one massaging element consisting of a massaging finger (21), each finger having a work head (22) intended to come into contact with the face, and each finger being able to move between:
- a position of retraction (R) in which the work head (22) is located below the pressure surface (S) toward the interior of the massaging head (1),
- a position of extension (E) in which the work head (22) is located above the pressure surface (S) toward the exterior of the massaging head (1),
- means of manoeuvring (25) each massaging finger (21) to be connected to the transmission mechanism and designed to move each of the massaging fingers (21) between its positions of extension (E) and retraction (R) in an alternating manner.

4. A massaging appliance (1) for the skin, comprising:
- a body (2) that includes driving means (3),
- at least one type of massaging head (4, 5, 6, 7, 8, ta, bi, ja) that includes at least two massaging elements,
- a transmission mechanism that activates the massaging elements via the propulsion of the driving means,
- means of attachment (9) that are configured to attach, in a removable manner, the at least one type of massaging head to the body,
- means of distinguishing (146) the type of massaging head attached to the body,
**characterized in that** control means (161) of said massaging appliance which, depending on the type of massaging head distinguished, are configured to act upon the driving means (3) so as to control the movement of at least two elements in the massaging head (4, 5, 6, 7, 8, ta, bi, ja), said control means (161) being configured to modify the activating of the driving means so as to adapt the activation speed of the massaging elements of the massaging head,
the massaging appliance (1) comprising a massaging head (1) that includes:
- a pressure element (20) that is intended to come into contact with the face and that forms a pressure crown (21) defining, on the one hand, a pressure surface (S) within a pressure plane (P) and, on the other hand, a work zone (Z) located inside the pressure crown,
- inside the pressure crown and in the work zone (Z) at least one massaging element consisting of a work head (22) with a work surface protruding from the pressure plane (P),
- manoeuvring means (25) to be connected to the transmission mechanism and designed to spin each work head (22) along at least one axis of rotation (Δ, Δ') offset from the centre of the corresponding work surface (T).

5. The massaging appliance (1) according to one of claims 1 to 4, in which the control means (161) of said massaging appliance (1) are configured to act upon the driving means (3) so as to control, in coordinated manner, a movement of said at least two massaging elements (4, 5, 6, 7, 8, ta, bi, ja).

6. The massaging appliance (1) according to one of claims 1 to 4, in which the control means (161) of said massaging appliance (1) are configured to act upon the driving means (3) so as to activate the spinning of each of said at last two massaging elements (4, 5, 6, 7, 8, ta, bi, ja).

7. The massaging appliance (1) according to one of the preceding claims, in which the massaging elements are chosen from among the set consisting of: massaging tip, massaging finger, massaging roller, work head, massaging ball.

8. The massaging appliance (1) according to one of the preceding claims, in which the control means (161) of said massaging appliance (1) are configured to act upon the driving means (3) so as to control at least one of the following parameters of said at least two massaging elements: spin speed, spin direction, oscillation frequency.

9. The massaging appliance (1) according to one of the preceding claims with means of emitting waves (106) and in which the control means (161) are configured to act upon the wave-emitting means (106) according to the type of massaging head (4, 5, 6, 7, 8, ta, bi, ja) attached to the body (2).

10. The massaging appliance (1) according to the preceding claim, in which the wave-emitting means (106) are means of emitting light waves among at least one of the following wavelengths: visible wavelengths including red, orange, and infrared wavelength.

11. The massaging appliance (1) according to claim 3 and claim 9 in which the control means (161) are configured, upon distinguishing the head, to act upon the visible orange light wave emitting system.

12. The massaging appliance (1) according to claim 4 and claim 9, in which the control means (161) are configured, upon distinguishing the head, to act upon the infrared light wave emitting system.

13. The massaging appliance (1) according to one of claims 9 or 10, wherein the control means (161) are configured to act upon the wave-emitting system (106) and modify the wavelength(s) and/or intensity and/or frequency of wave emission depending on the type of massaging head (4, 5, 6, 7, 8, ta, bi, ja) attached to the body (2).

14. The massaging appliance (1) according to one of claims 9, 10 or 13 comprising wave emitting means (106) located on the body (2) and means of transferring (108) the waves from the wave-emitting system to the massaging head (4, 5, 6, 7, 8, ta, bi, ja) .

15. The massaging appliance (1) according to one of claims 9, 10, 13 or 14, in which the wave-emitting means (106) include electroluminescent diodes.

16. The massaging appliance (1) according to one of the preceding claims, in which the control means (161) are configured to act simultaneously upon the wave-emitting system (106) and on the driving means (3) so as to control the movement of at least two elements of the massaging head (4, 5, 6, 7, 8, ta, bi, ja) and, to do so, depending on the type of massaging head distinguished, to establish at least two different phases of skin treatment during the treatment.

17. The massaging appliance (1) according to one of the preceding claims, wherein the distinguishing means (13, 146) consist of mechanical, magnetic or optical sensors (151, 152, 164, 165, 169, 170) located on at least one type of massaging head (147) and the body (2), and are configured to transmit information to the control means (161) depending on the type of massaging head attached to the body.

18. The massaging appliance (1) according to one of the preceding claims having a cosmetic product dispensing system (77) located on the body and means of transfer (90) of the product from the body (2) to the massaging head (4, 5, 6, 7, 8, ta, bi, ja).

19. The massaging appliance (1) according to the preceding claim, wherein the control means (161) are configured to act upon the cosmetic product dispensing system and modify at least the dispensing of the cosmetic product depending on the type of massaging head (4, 5, 6, 7, 8, ta, bi, ja) attached to the body (2) .

20. The massaging appliance (1) according to one of the preceding claims having a transcutaneous ionophoresis treatment device which is configured to transmit to the skin, while said massaging appliance is applied, a current that improves and/or accelerates the penetration of a cosmetic product.
